# FASCICULE DE BREVET EUROPEEN

(11) **EP 3 024 927 B1**
(45) Date de publication et mention de la délivrance du brevet: **27.12.2017**
(21) Numéro de dépôt: 14742242.2
(22) Date de dépôt: 24.07.2014
(51) Int. Cl.: C12N 5/077, C12N 7/00

(54) **PROCEDE DE SÉLECTION D'UNE LIGNEE CELLULAIRE PERMISSIVE POUR LA REPLICATION DE VIRUS AVIAIRES**
VERFAHREN ZUR AUSWAHL EINER PERMISSIVEN ZELLLINIE ZUR REPLIKATION VON VOGELGRIPPEVIREN
METHOD FOR SELECTING A PERMISSIVE CELL LINE FOR REPLICATING AVIAN VIRUSES

(30) Priorité: 25.07.2013 FR 1357346
(43) Date de publication de la demande: 01.06.2016
(73) Titulaire: Institut National De La Recherche Agronomique, 75007 Paris (FR)
(72) Inventeur: VAUTHEROT, Jean-François, F-37380 Monnaie (FR); PAIN, Bertrand, F-63830 Nohanent (FR); DENESVRE, Caroline, F-37000 Tours (FR); FRAGNET - TRAPP, Laetitia, F-37110 La Ferriere (FR)
(74) Mandataire: Regimbeau
(86) Numéro de dépôt international: PCT/EP2014/065946
(87) Numéro de publication internationale: WO 2015/011238

(56) Documents cités:
- EP-A1- 2 572 728
- EP-A2- 0 775 743
- WO-A1-2007/110341
- GEERLIGS H ET AL: "Efficacy and safety of cell associated vaccines against Marek's disease virus grown in a continuous cell line from chickens", VACCINE, ELSEVIER LTD, GB, vol. 26, no. 44, 16 octobre 2008 (2008-10-16), pages 5595-5600, XP025469577, ISSN: 0264-410X, DOI: 10.1016/J.VACCINE.2008.07.080 [extrait le 2008-08-14] cité dans la demande
- BROWN S W ET AL: "The avian EB66(R) cell line, application to vaccines, and therapeutic protein production", PDA JOURNAL OF PHARMACEUTICAL SCIENCE AND TECHNOLOGY>BULL PARENTER DRUG ASSOC, PARENTERAL DRUG ASSOCIATION, vol. 64, no. 5, 1 septembre 2010 (2010-09-01), pages 419-425, XP008132170, ISSN: 1079-7440
- LEE JEONGYOON ET AL: "Establishment of an immortal chicken embryo liver-derived cell line", POULTRY SCIENCE, vol. 92, no. 6, juin 2013 (2013-06), pages 1604-1612, XP008168331, DOI: 10.3382/PS.2012-02582
- SCHAEFER-KLEIN J ET AL: "The EV-O-Derived Cell Line DF-1 Supports the Efficient Replication of Avian Leukosis-Sarcoma Viruses and Vectors", VIROLOGY, ELSEVIER, AMSTERDAM, NL, vol. 248, no. 2, 1 septembre 1998 (1998-09-01), pages 305-311, XP004445698, ISSN: 0042-6822, DOI: 10.1006/VIRO.1998.9291
- C. DENESVRE ET AL: "Morphogenesis of a Highly Replicative EGFPVP22 Recombinant Marek's Disease Virus in Cell Culture", JOURNAL OF VIROLOGY, vol. 81, no. 22, 15 novembre 2007 (2007-11-15), pages 12348-12359, XP055109231, ISSN: 0022-538X, DOI: 10.1128/JVI.01177-07 cité dans la demande
- KITAGAWA R ET AL: "Hexamethylene bisacetamide can convert nonpermissive human cells to a permissive state for expressing the major immediate-early genes of human cytomegalovirus by up-regulating NF-kappaB activity", VIROLOGY, ELSEVIER, AMSTERDAM, NL, vol. 383, no. 2, 20 janvier 2009 (2009-01-20), pages 195-206, XP025865832, ISSN: 0042-6822, DOI: 10.1016/J.VIROL.2008.10.028 [extrait le 2008-11-22]
- PRESTON C M ET AL: "Cytodifferentiating Agents Affect the Replication of Herpes Simplex Virus Type 1 in the Absence of Functional VP16", VIROLOGY, ELSEVIER, AMSTERDAM, NL, vol. 249, no. 2, 30 septembre 1998 (1998-09-30), pages 418-426, XP004445658, ISSN: 0042-6822, DOI: 10.1006/VIRO.1998.9314 cité dans la demande

## Description

### DOMAINE DE L'INVENTION

La présente invention concerne un procédé de sélection d'une lignée cellulaire permissive pour la réplication de virus aviaires, à partir de cellules souches embryonnaires aviaires.

### INTRODUCTION

Les élevages avicoles sont affectés ou sous la menace de plusieurs affections virales dont les effets peuvent être directs (pathologie viro-induite) ou indirects (immunodépression faisant le lit d'autres infections virales, bactériennes ou parasitaires).

La vaccination, largement pratiquée chez les principales espèces aviaires de rente (poulet, dinde, pintade, caille, oie, canard...) pose cependant 2 problèmes :
- Les coûts de production des vaccins doivent rester compatibles avec un élevage ne dégageant qu'une faible marge de bénéfices pour l'éleveur. Bien que le marché soit considérable en terme d'individus vaccinés (exemple : 44 millions de poules pondeuses en France en 2011), les vaccins ne peuvent être commercialisés à des prix dépassant les quelques centimes d'€ / dose.
- La biosécurité doit être assurée, surtout lors de l'utilisation de vaccins vivants atténués, vecteurs potentiels d'agents pathogènes contaminants. Il convient de se souvenir que les animaux reproducteurs sont vaccinés et qu'une contamination accidentelle du sommet de la pyramide de sélection aurait des effets dramatiques sur l'ensemble de la chaine de production.

Parmi les nombreuses pathologies d'origine virales, la maladie de Marek (Marek's Disease - MD) est une maladie infectieuse lympho-proliférative affectant les cellules T chez le poulet. Les formes cliniques de la MD affectent principalement la poule (Gallus gallus) mais d'autres espèces d'oiseaux de l'ordre des Galliformes peuvent également être infectés et exprimer des symptômes (caille ou dinde). Cette maladie est progressivement apparue comme une contrainte majeure pour la production avicole mondiale au cours des années 1960. La maladie de Marek, considérée comme la deuxième maladie infectieuse pour les poules pondeuses, a un impact global estimé à 1 milliard de $/an, ces pertes étant liées à la pathologie et aux coûts de vaccinations (Chiffre FAO, 2002).

La maladie de Marek est due à un herpesvirus de la sous-famille des alpha-herpesvirinae, du genre Mardivirus, nommé le virus de la maladie de Marek (MDV) ou Gallid herpesvirus type 2 (GaHV-2). Le genre Mardivirus compte quatre autres virus aviaires: Gallid herpesvirus type 3 (GaHV-3), Meleagrid herpesvirus (MeHV - HVT pour Herpes Virus of Turkey) qui affecte plus spécifiquement les dindes, Colombid herpesvirus 1 (CoHV-1) qui touche particulièrement les Colombiformes, et Anatid herpesvirus 1 (AHV-1) qui touche particulièrement les Anatidés (canards, oies).

Le MDV se propage rapidement à travers les élevages par contact direct des animaux sains avec des poulets infectés, ou par exposition à des locaux, litières, poussières contaminés par les débris de phanères issus d'animaux infectés. L'entrée du virus a lieu au niveau des voies respiratoires où les lymphocytes B pulmonaires jouent un rôle important pour la progression de la maladie.

Des formes suraigües de la maladie sont apparues dans les années 1990, associées à l'émergence de virus très pathogènes.

Depuis les années 1970, des vaccins permettent de contrôler la maladie de Marek dans les élevages avicoles. Seuls les vaccins vivants sont efficaces et il existe 2 types de vaccins, ceux obtenus par atténuation du GaHV-2 (homologues) ou ceux dérivant de souches naturellement apathogènes, MeHV-1 ou GaHV-3 (hétérologues). Les préparations vaccinales dérivant du MeHV-1 sont administrées *in ovo* aux embryons de 17-18 jours et les vaccins homologues sont injectés aux poussins à l'éclosion. L'infectiosité du virus GaHV-2 est associée aux cellules infectées vivantes : il n'y a pas de virions libres détectables dans les surnageants ou dans les lysats cellulaires (Churchill, 1968). La propagation de l'infection par GaHV-2 nécessite donc *in vitro* la co-culture de cellules infectées avec des cellules naïves, ou, *in vivo,* l'administration, par injection aux animaux, de cellules infectées vivantes. Les vaccins GaHV-2 actuels sont donc constitués de cellules infectées en culture cellulaire et congelées.

### ETAT DE LA TECHNIQUE

L'étude des relations entre le virus et les cellules ne peut se faire qu'avec des cellules dites permissives et pour le MDV, la réplication est restreinte aux cellules aviaires. Une cellule est dite permissive lorsqu'elle permet au virus de se multiplier et de produire de nouveaux virions infectieux. Le génotype et l'état de différenciation d'une cellule sont les deux principaux facteurs déterminant de la permissivité d'une cellule à l'infection par un virus.

La propagation des Mardivirus en culture cellulaire est actuellement restreinte aux cellules aviaires d'explantation primaire ou secondaire. Des niveaux de réplication satisfaisants sont couramment obtenus sur 4 types cellulaires différents : des fibroblastes d'embryons de poule (CEF), des fibroblastes d'embryons de canard (DEF) (Solomon et al., 1968), des cellules de rein de poulet (CKC) (Churchill, 1968) ou des cellules de peau d'embryons de poule (CESkC) (Dorange *et al.,* 2000).

Le faible nombre de lignées cellulaires aviaires permissives permettant une réplication efficace des virus aviaires pose un problème tant aux scientifiques qu'aux industriels producteurs de vaccins.

Ce problème se pose avec une grande acuité pour les Mardivirus, pour lesquels aucune lignée aviaire non transformée n'est disponible à ce jour permettant une réplication efficace, c'est-à-dire productrice, de tous les Mardivirus. Les vaccins sont donc encore exclusivement produits sur cellules aviaires primaires obtenues par mise en culture de cellules embryonnaires ou par dissociation enzymatique d'organes explantés d'oiseaux (Galliformes ou Ansériformes). Cela suppose des coûts importants, des circuits de production et de contrôles complexes permettant de certifier l'absence d'agents contaminants pour chaque cycle de production. Jusqu'à présent les essais d'isolement de lignées cellulaires à partir de cellules explantées d'organes ou d'embryons se sont avérés peu fructueux.

A l'heure actuelle seule une lignée dérivée de la lignée DF-1 (JB-J1) semble pouvoir remplir les critères essentiels pour une production industrielle de virus vaccins (Geerligs *et al.,* 2008) sans qu'il soit démontré que cette lignée puisse servir de substrat à la réplication des virus non "adaptés" à la culture cellulaire (virus « sauvages » récupérés sur un animal infecté). Pour ces virus « sauvages », les seuls résultats rapportés concernent l'utilisation d'une lignée de cellules transformées de caille (QM7) surexprimant un gène de glycoprotéine virale, gE (Schumacher et al., 2002). Ces cellules nommées SOgE supportent la croissance de souches virales MDV-1 à un niveau comparable à celui observé sur des cultures primaires de cellules embryonnaires. Néanmoins, cette dernière lignée cellulaire dérive d'une lignée de cellules transformées c'est-à-dire capables de se multiplier indéfiniment, avec, chez l'animal, un pouvoir tumorigène potentiel lié au processus d'immortalisation; il est donc dangereux d'utiliser de telles cellules pour la préparation de préparations vaccinales destinées à être injectées à des poulets, car leur prolifération n'est pas contrôlée. De plus, les cellules SOgE sont des Organismes Génétiquement Modifiés dont l'utilisation est strictement réglementée, et qui ne seront probablement pas autorisés pour une utilisation dans des préparations vaccinales.

Il est à noter que les cellules DF1 (et donc les JB-J1 qui en sont dérivées) sont obtenues par un établissement spontané de fibroblastes embryonnaires primaires obtenus à partir d'un embryon incubé pendant 10 jours, comme décrit dans le document WO98/006824. Ces cellules sont donc dérivées de cellules somatiques déjà engagées dans une voie de différenciation.

De la même façon, les cellules CHCC-OU2, décrites dans la demande de brevet EP 775743 sont dérivées de cellules d'embryons incubés pendant plusieurs jours. Ces cellules sont établies en lignée par l'action immortalisante d'un traitement chimique des cellules primaires avec le N-Methyl-N'-nitro N-nitrosoguanidine (MNNG), comme le décrit la publication d'Ogura et Fujiwara (1987). L'action d'un tel traitement sur les cellules aviaires est connue et aboutit à l'obtention de lignée immortalisée, mais présentant des propriétés oncogènes. Ainsi, par exemple, les cellules fibroblastiques de caille QT6 qui ont également été obtenues par traitement chimique ont dé propriétés tumorigènes (Moscovici et al., 1977).
De plus, la permissivité des cellules CHCC-OU2 à répliquer les mardivirus n'est pas établie. En l'état, les cellules CHCC-OU2 ne constituent pas un système répertorié comme permissif aux Mardivirus. D'ailleurs, la lignée dérivée de CHCC-OU2 décrite dans la demande de brevet EP 775 743 est une lignée infectée de façon chronique par un virus MDV, et non pas une lignée cellulaire permissive pour la réplication des Mardivirus.

De la même façon, Lee et al. (2013) décrivent des cellules obtenues par immortalisation de cellules hépatiques d'un embryon déjà incubé, comme l'obtention de la lignée LMH, elle-même dérivée de cellules hépatiques embryonnaires établies à la suite d'un traitement chimique (Kawaguchi et al., 1987) Ces cellules comme dans le cas des cellules QT6 sont fortement tumorigènes. Elles ne sont pas décrites comme fibroblastiques, et sont peu ou pas permissives aux mardivirus (titres infectieux = 10 unités formant plaques (ufp) contre 500 000 pour les ESCDL-1) : il n'est donc pas établi que les cellules soient capables de réellement répliquer le virus, les 10 ufp résiduelles pouvant être dues à la persistance de cellules DF-1 (inoculum viral constitué de cellules infectées) lors de la co-culture avec les cellules CEL.im. Le document SCHAEFER-KLEIN J ET AL: "The EV-O-Derived Cell Line DF-1 Supports the Efficient Replication of Avian Leukosis-Sarcoma Viruses and Vectors", VIROLOGY, ELSEVIER, AMSTERDAM, NL, vol. 248, no. 2, 1 septembre 1998 (1998-09-01), pages 305-311 décrit que la lignée de cellules DF-1, une lignée continue non-transformée de cellules fibroblastiques d'un embryon de poulet EV-O est susceptible à l'infection par le virus aviaire ASLV.

Le document WO2007110341 décrit un procédé de préparation de cellules aviaires différenciées à partir de cellules souches aviaires cultivées dans un milieu de culture approprié, caractérisé en ce qu'il comprend une étape d'induction de la différenciation des cellules souches par inhibition de l'expression ou de l'activité d'un gène exprimé dans lesdites cellules souches, sélectionné parmi les gènes 1P06, Nanog et Eomes (revendication 1). Selon un mode de réalisation préféré, la-dite inhibition y est réalisée par introduction d'un ARN interférent (siRNA) (revendication 3).

Le document KITAGAWA R ET AL: "Hexamethylene bisacetamide can convert nonpermissive human cells to a permissive state for expressing the major immediate-early genes of human cytomegalovirus by up-regulating NF-kappaB activity", VIROLOGY, ELSEVIER, AMSTERDAM, NL, vol. 383, no. 2, 20 janvier 2009 (2009-01-20), pages 195-206 décrit l'induction par traitement de cellules avec du HMBA d'un état permissif à l'infection par le cytomégalovirus humain.

Enfin, les cellules EB66, décrites dans la demande de brevet EP 2 572 728 et dans Brown SW et al, PDA Journal of Pharmaceutical Science and Technology, vol. 64, (2010-09-01), pages 419-425 sont bien d'origine embryonnaire, comme les cellules cES à l'origine des cellules ESCDL1 de la présenet invention. Toutefois, elles conservent les caractéristiques de cellules ES notamment une expression des marqueurs cPOUV/OCT4 et NANOG. De plus, ces cellules EB66 ne sont pas permissives aux Mardivirus.

### EXPOSE DE L'INVENTION

La présente invention est relative à un procédé permettant l'obtention d'une lignée cellulaire non transformée, permissive à la réplication des virus aviaires, pouvant être utilisée dans des préparations vaccinales.

L'invention est relative à un procédé d'induction de différenciation des cellules souches aviaires, aboutissant à l'obtention d'une lignée non transformée, dérivée de cellules souches embryonnaires (ES), n'exprimant plus les marqueurs caractéristiques des cellules souches (cPOUV et NANOG), stable phénotypiquement sur au moins 60 passages après établissement, et permettant la réplication de virus aviaires.

Le procédé comprend au moins les étapes suivantes :
a) Mise en culture de cellules souches embryonnaires aviaires en présence d'un stroma pendant au moins 3 jours ;
b) Culture des cellules issues de l'une des étapes a), ou c), ou d) dans un milieu à faible concentration de sérum pendant au moins 2 jours ;
c) Culture des cellules issues de l'une des étape a), oub), ou d) dans un milieu à faible concentration de sérum comprenant entre 1 et 10 mM de hexaméthylène bisacétamide (HMBA), pendant au moins 2 jours ;
d) Culture des cellules issues de l'une des étapes a), ou b), ou c) dans un milieu à faible concentration de sérum pendant au moins 10 jours ;
e) Culture ou congélation d'une lignée cellulaire aviaire résultant des étapes a), b), c) et d) permettant la réplication de virus aviaires.

L'invention est également relative à la lignée cellulaire telle qu'obtenue par le procédé ci-dessus et caractérisée en ce qu'elle exprime les marqueurs suivants : CD44+, intégrine beta1, collagène 1 et OLFM3.

La présente invention est aussi relative à l'utilisation de la lignée cellulaire décrite ci-dessus comme substrat pour la production *in vitro* de virus aviaires ou pour titrer des virus aviaires.

Un autre objet de la présente invention concerne par ailleurs un procédé de préparation *in vitro* d'une préparation vaccinale qui comprend la mise en culture de la lignée telle que décrite ci-dessus et son infection par au moins un virus aviaire, ainsi que la préparation vaccinale proprement dite qui comprend une lignée cellulaire telle que décrite ci-dessus, infectée par au moins un virus aviaire.

### DESCRIPTION DETAILLEE DE L'INVENTION

La présente invention est relative à un procédé d'obtention d'une lignée cellulaire aviaire non transformée permettant la réplication des virus aviaires *in vitro,* comprenant au moins les étapes suivantes :
A) Mise en culture de cellules souches embryonnaires aviaires en présence d'un stroma pendant au moins 3 jours ;
B) Culture dans un milieu à faible concentration de sérum pendant au moins 2 jours ;
C) Culture dans un milieu à faible concentration de sérum comprenant entre 1 et 10 mM de hexaméthylène bisacétamide (HMBA), pendant au moins 2 jours ;
D) Culture dans un milieu à faible concentration de sérum pendant au moins 10 jours ;
E) Culture ou congélation d'une lignée cellulaire aviaire permettant la réplication de virus aviaires.

Le procédé selon l'invention a comme « produit d'origine », ou cellules de départ ou cellules initiales, des cellules souches embryonnaires aviaires (cellules ES), exprimant spécifiquement les marqueurs cPOUV et NANOG comme décrit dans Lavial et al. 2007.

La différenciation des cellules ES aviaires par exposition à des concentrations réduites en sérum se traduit par un changement morphologique (augmentation de taille des cellules et augmentation du rapport cytoplasme/noyau) et l'acquisition d'une faible permissivité à la réplication des virus « adaptés » à la culture sur cellule.

L'établissement d'une lignée permissive à partir de cellules ES aviaires a été conduit selon une logique d'induction reposant sur l'exposition à au moins une matrice extracellulaire complexe et à l'action cytodifférenciante du HMBA.

La présente invention permet d'obtenir des cellules permissives à la réplication des virus aviaires qui sont établies en lignée, sans faire appel à la transformation par un oncogène ou un procédé d'immortalisation à l'aide d'un agent extérieur tel qu'un agent chimique, contrairement aux cellules de l'art antérieur, dont en particulier les cellules CHCC-OU2. Les cellules de l'invention sont établies par différenciation, notamment à l'aide d'un traitement chimique cyto-différenciant (HMBA), et non pas par transformation ou par immortalisation. L'établissement de ces cellules permissives est donc non spontané, contrairement à certaines cellules de l'art antérieur comme les cellules DF1, car elles dérivent de cellules déjà établies (cellules ES) et non pas d'une cellule primaire somatique.

### Définitions

D'une manière générale, dans le cadre de la présente invention, l'expression « réplication des virus aviaires » doit se comprendre comme signifiant une réplication efficace et productive de virus. Ainsi, on entend par « lignée ou cellules permissives à la réplication des virus aviaires », une lignée ou des cellules capables d'accomplir l'ensemble des étapes du cycle viral aboutissant à la production d'une progénie virale constituée de virions infectieux, et en particulier de Mardivirus et Birnavirus (dans le cas des Mardivirus, les virions infectieux sont associés aux cellules au sein d'un inoculum). Comme indiqué plus haut, une cellule permissive permet l'entrée et la réplication productive du virus. La permissivité à la réplication des virus aviaires est variable d'un virus à l'autre, ce que l'homme de métier saura apprécier au cas par cas en fonction des virus considéré. L'homme du métier saura ainsi déterminer le facteur multiplicatif recherché pour chaque particule initiale selon le type de virus testé.

Le terme « mise en culture dans un milieu approprié » est aisément compréhensible pour l'homme du métier spécialiste en culture cellulaire. Il désigne le fait de faire croître des cellules *in vitro,* dans un milieu nutritif approprié, le plus souvent liquide, dans des conditions optimisées pour la croissance des cellules, notamment en termes de température et de concentration contrôlée en CO2. Le « milieu approprié » désigne un milieu de culture classique, adapté aux cellules aviaires, comprenant du sérum.

Le terme « sérum » désigne le liquide sanguin débarrassé de ses cellules et des protéines de la coagulation. C'est le liquide surnageant obtenu après la centrifugation du sang, sans inhibiteur de la coagulation. Ce liquide contient un grand nombre de nutriments (acides aminés, vitamines...), des protéines solubles (anticorps, albumine, cytokines, facteurs de croissance, etc.) ainsi que divers ions (sodium, chlorure, etc.). Dans la présente demande, le terme « sérum » désigne aussi bien le sérum de veau foetal, classiquement utilisé en culture cellulaire, que le sérum de poulet. Une combinaison des deux peut être utilisée, pour associer les avantages de chaque sérum. Le sérum est ajouté au milieu de culture selon un pourcentage du volume total, i.e. environ 100 ml de sérum dans un litre de milieu total est exprimé comme une concentration de « 10% de sérum ».

L'expression « faible teneur ou concentration en sérum » désigne un pourcentage de sérum inférieur à 5%, en particulier 0,5%, 1%, 1,5%, 2%, 2,5%, 3%, ou 4% de sérum.

Selon l'invention, le terme « stroma » désigne une matrice extracellulaire complexe dérivée de cellules. Par exemple, le stroma selon l'invention peut être préparé à partir de cellules primaires de peau de poulet selon le protocole décrit par Coraux (Coraux et al., 2003) ; il est à noter que dans cette publication, ce stroma avait été utilisé pour la différenciation de cellules souches embryonnaires de souris en tissu épidermique. Le stroma peut aussi dériver de cellules permissives aux virus aviaires (*Stromal cell-Derived Inducing Activity -* SDIA - (Kawasaki et al., 2000) ou encore de tout type de cellules non permissives aux virus. Parmi ces dernières sont compris dans une liste non exhaustive des fibroblastes primaires embryonnaires de souris (MEFs), couramment utilisés comme tapis nourricier après inactivation par action de la mitomycine ou irradiation, des fibroblastes de souris établis en lignées comme les cellules STO (ATCC CRL 1503), permettant le maintien des cellules ES, les cellules 3T3-J2 (Panacchia et al.2010), les cellules de stroma de moelle comme les cellules PA6 (aussi appelé MC3T3 G2) permettant la différenciation neurectodermique (Correia et al., 2008), les cellules de stroma de moelle comme les cellules OP9 (ATCC CRL2749) (Nakano et al., 1994) permettant l'induction de la différenciation lymphopoiétique, ou des kératinocytes humains ou animaux ou tout type cellulaire présentant un intérêt pour l'homme de l'art. Dans le cadre de l'invention, l'homme du métier saura adapter le type de stroma au type de différenciation.

Le facteur de différenciation hexaméthylène bisacétamide (HMBA - de formule chimique CH₃CONH(CH₂)₆NHCOCH₃) est connu pour son action cyto-différenciante vis à vis des cellules murines de lignée érythroleucémique dans lesquelles la différenciation est associée à une perte des capacités à proliférer (Marks & Rifkind, 1989). Dans le contexte des infections par les herpesvirus, l'induction par le HMBA confère aux cellules soit une permissivité accrue permettant la réplication de certains virus défectifs (Preston & McFarlane, 1998), soit une expression accrue des facteurs de transcription PBX/HOX favorisant la réplication virale (Storlie *et al.,* 2006). Le HMBA est couramment utilisé, à des doses de 1 à 10 mM, en tant qu'inducteur de différenciation pour les cellules primaires de peau d'embryon de poule servant de substrat à la réplication des Mardivirus (Denesvre *et al.,* 2007).

Le terme « passage » désigne la mise en culture de cellules ayant atteint un taux d'occupation maximum de leur support. Les cellules sont détachées de leur support par action rapide d'une enzyme ou d'un cocktail d'enzymes protéasiques (trypsine, pronase, dispase, collagénase) dissociant les cellules entre elles et favorisant leur détachement de la matrice extracellulaire (action de « trypsination ») et sont diluées dans du milieu de culture avant d'être ensemencées sur un nouveau support pour une nouvelle mise en culture de quelques jours. Classiquement, les cellules détachées de leur support sont dénombrées avant d'être ensemencées à un certain ratio de cellules/cm² en boites de culture prétraitées durant au moins 20 minutes par une solution de gélatine bovine à 0,1% en tampon phosphate (PBS), afin d'apporter aux cellules transférées une matrice extracellulaire favorisant leur adhésion sur le support plastique.

Les différentes étapes a), b), c) et d) sont pratiquées pendant le temps nécessaire. En particulier, l'homme du métier suivra la différenciation des cellules en visualisant, à l'aide d'un microscope, leur croissance et leur viabilité, et sera à même de décider si l'étape doit se poursuivre au-delà du temps minimum indiqué.

L'étape a) de culture en présence d'un stroma sera réalisée pendant au moins 2 jours, préférentiellement pendant au moins 3 jours, notamment 3 jours, plus préférentiellement pendant 3 à 5 jours.

L'étape b) de culture dans un milieu à faible concentration de sérum sera réalisée pendant au moins 2 jours, préférentiellement pendant 3 jours.

L'étape c) de culture dans un milieu comprenant du HMBA sera réalisée pendant au moins 2 jours, préférentiellement pendant 3 jours.

L'étape d) de culture dans un milieu à faible concentration de sérum sera réalisée pendant au moins 10 jours, préférentiellement pendant 15 jours.

De manière préférée, après les différentes étapes b), c) et d), le procédé comprend de plus des étapes de culture des cellules dans un milieu de croissance approprié, en particulier dans un milieu comprenant plus de 5% de sérum, et préférentiellement environ 10% de sérum.

La dernière étape e) correspond à l'obtention de la lignée cellulaire permissive à la réplication des virus, qui pourra selon les cas être utilisée de suite pour la réplication de virus ou pourra être congelée selon la technique bien connue de l'homme du métier.

Le procédé peut être détaillé comme indiqué ci-dessous :
a) Mise en culture de cellules souches embryonnaires aviaires en présence d'un stroma pendant 4 jours ;
b) Culture des cellules dans un milieu à faible concentration de sérum pendant 3 jours ;
c) Culture des cellules dans un milieu contenant 10% de sérum pendant 10 jours ;
d) Culture des cellules dans un milieu à faible concentration de sérum comprenant entre 1 et 10 mM de HMBA (hexaméthylène bisacétamide), pendant 3 jours ;
e) Culture des cellules dans un milieu à faible concentration de sérum pendant 15 jours ;
f) Culture ou congélation d'une lignée cellulaire aviaire permettant la réplication de virus aviaires.

De manière encore plus détaillée, le procédé peut comprendre les étapes suivantes :
a) Mise en culture de cellules souches embryonnaires aviaires en présence d'un stroma pendant 4 jours ;
b) Culture des cellules dans un milieu à faible concentration de sérum pendant 3 jours ;
c) Culture des cellules dans un milieu contenant 10% de sérum pendant 10 jours et passage par trypsination;
d) Culture des cellules dans un milieu à faible concentration de sérum comprenant entre 1 et 10 mM de HMBA (hexaméthylène bisacétamide), pendant 3 jours ;
e) Passage des cellules par trypsination en milieu contenant 10% de sérum pendant 3 jours
f) Culture des cellules dans un milieu à faible concentration de sérum pendant 15 jours ;
g) Passage des cellules par trypsination en milieu contenant 10% de sérum pendant 12 jours ;
h) Culture ou congélation d'une lignée cellulaire aviaire ayant un phénotype stable pendant au moins 60 passages dans un milieu de culture approprié et permettant la réplication de virus aviaires.
Toutes les étapes a) à d), a) à f) et a) à h) du procédé de l'invention telles que décrites ci-dessus peuvent être réalisées dans n'importe quel ordre, avantageusement elles sont réalisées dans l'ordre de a) à d), de a) à f) et de a) à h). Par exemple, lorsque les étapes a) à d) sont réalisées dans cet ordre, le procédé de l'invention comprend au moins les étapes suivantes :
A) Mise en culture de cellules souches embryonnaires aviaires en présence d'un stroma pendant au moins 3 jours ;
B) Culture des cellules issues de l'étape A), dans un milieu à faible concentration de sérum pendant au moins 2 jours ;
C) Culture des cellules issues de l'étape B), dans un milieu à faible concentration de sérum comprenant entre 1 et 10 mM de hexaméthylène bisacétamide (HMBA), pendant au moins 2 jours ;
D) Culture des cellules issues de l'étape C), dans un milieu à faible concentration de sérum pendant au moins 10 jours ;
E) Culture des cellules issues de l'étape D), ou congélation d'une lignée cellulaire aviaire permettant la réplication de virus aviaires.

Le produit résultant du procédé consiste en des cellules permissives pour la réplication des virus aviaires, pouvant être maintenues avec le même phénotype pendant au moins 60 passages dans un milieu de culture approprié, et n'exprimant plus les marqueurs cPOUV et NANOG caractéristiques des cellules ES.

Ces cellules ont été baptisées « ESCDL-1 » pour « Embryonic Stem Cell Derived Line 1 ». Les ESCDL-1 sont des cellules adhérentes, d'aspect fibroblastique, c'est-à-dire plus étalées et moins étirées qu'un fibroblaste primaire. Elles diffèrent de la lignée de cellules ES par leur morphologie, leurs caractéristiques de croissance, et l'extinction de l'expression de marqueurs de cellules ES.

Cette dernière caractéristique permet aussi de les distinguer des cellules de l'art antérieur, comme les cellules EB 66 (voir EP 2572 728) ou les cellules DF1, LMH, CEF et cES (BP25) (voir l'exemple 2 de la présente demande), chez qui l'expression de marqueurs des cellules ES est maintenue. Ainsi, les cellules ESCDL-1 de l'invention n'expriment pas l'un au moins des marqueurs suivants : cPOUV/OCT4, NANOG, SSEA1, EMA1, IXF1, KRT8 et KRT19. Plus préférentiellement, elles n'expriment pas au moins deux, au moins trois, au moins quatre, au moins cinq, ou au moins six de ces marqueurs. Encore plus préférentiellement, elles n'expriment aucun de ces marqueurs.

Par ailleurs, les cellules ESCDL-1 de l'invention expriment fortement le gène OLFM3 et les gènes WISP1, THBS2 et EDN2 à des niveaux comparables à ceux observés dans des fibroblastes primaires.

Les cellules de l'invention présentent une activité télomérase faible comparée à celle des cellules ES. Les cellules ESCDL1 sont des cellules non transformées et établies en lignée qui prolifèrent, mais qui sont non tumorigènes contrairement aux lignées établies chimiquement ou par l'action d'un immortalisant exogène. La capacité de prolifération des cellules ESCDL-1 (20% de cellules en phase S) est supérieure aux cellules primaires de peau embryonnaire de poulet (CEPP) (3% de cellules en phase S). De plus, il est intéressant de souligner que les cellules ESCDL-1 sont sensibles aux inducteurs d'apoptose et présentent une forte propension à induire des processus autophagiques en milieu appauvri en facteurs de croissance ou en présence d'inducteur d'autophagie.

Le procédé selon l'invention permet d'obtenir une ligne cellulaire permissive pour la réplication de nombreux virus aviaires (notamment Herpesvirus, Poxvirus, Coronavirus, Birnavirus, Réo et Rotavirus) et en particulier pour les Mardivirus et le Birnavirus, agent de la bursite infectieuse (IBDV).

Selon un aspect préféré de l'invention, les cellules souches embryonnaires aviaires de départ ou initiales sont des cellules issues de poulet. Préférentiellement, les cellules ES sont issues d'un poulet de génotype Cou-nu hétérozygote (Na/na).

Plus préférentiellement, les cellules souches embryonnaires aviaires initiales sont des cellules isolées de blastoderme aux stades X à XIV (selon Eyal Giladi et Kovak, 1976). Contrairement à certaines cellules de l'art antérieur, la lignée cellulaire selon l'invention est d'origine embryonnaire non incubé puisqu'elle dérive de cellules souches embryonnaires, elles même obtenues à partir de la mise en culture de ces cellules de blastodermes non incubés.

Ces cellules souches embryonnaires aviaires initiales expriment de nombreux gènes marqueurs tels que ceux présentés dans la demande de brevet WO 2007/110343, et en particulier les gènes marqueurs cPOUV et NANOG.

Selon un aspect préféré de l'invention, la lignée cellulaire aviaire obtenue a un phénotype stable pendant au moins soixante passages successifs, préférentiellement au moins 70 passages, plus préférentiellement au moins 80 passages, à partir de son établissement.

Préférentiellement, les caractéristiques phénotypiques des cellules aviaires selon l'invention sont plus particulièrement les suivantes :
▪ Morphologie de type fibroblastique/mésenchymateuse ;
▪ Expression de CD44, intégrine beta1, et collagène 1 ;
▪ Non expression de marqueurs des cellules ES, dont, notamment, cPOUV et NANOG, KRT19 et KRT8, mais forte expression du gène OLFM3 et expression maintenue des gènes WISP1, THBS2 et EDN2 à des niveaux comparables à ceux observés dans des fibroblastes primaires ; et
▪ Permissivité à la réplication des Mardivirus et des Birnavirus aviaires.

Par cellule fibroblastique mésenchymateuse, on entend une cellule possédant une morphologie similaire à celle des cellules peu différenciées des tissus conjonctifs avec notamment de longs prolongements et un noyau ovoïde au centre de la cellule

Selon un autre aspect de l'invention, la lignée cellulaire aviaire obtenue peut être modifiée génétiquement pour exprimer des protéines d'intérêt. Les protéines d'intérêts peuvent être des protéines « rapporteurs » (protéines fluorescentes ; enzymes telles que les différentes formes de luciférases ou de phosphatases...), des enzymes de modification du génome (recombinases), des protéines virales autologues ou hétérologues pouvant intervenir en complémentation pour des virus dont certains gènes ont été délétés, ou encore des protéines cellulaires dont la surexpression pourrait avoir un impact sur la réplication virale.

Comme montré dans les exemples, même modifiée génétiquement comme décrit ci-dessus, la lignée cellulaire aviaire selon l'invention conserve sa permissivité aux virus aviaires.

La présente invention est également relative à une lignée cellulaire susceptible d'être obtenue par le procédé décrit ci-dessus, c'est-à-dire une lignée cellulaire non transformée permettant la réplication des virus aviaires *in vitro,* en particulier à la fois des Mardivirus et des Birnavirus.

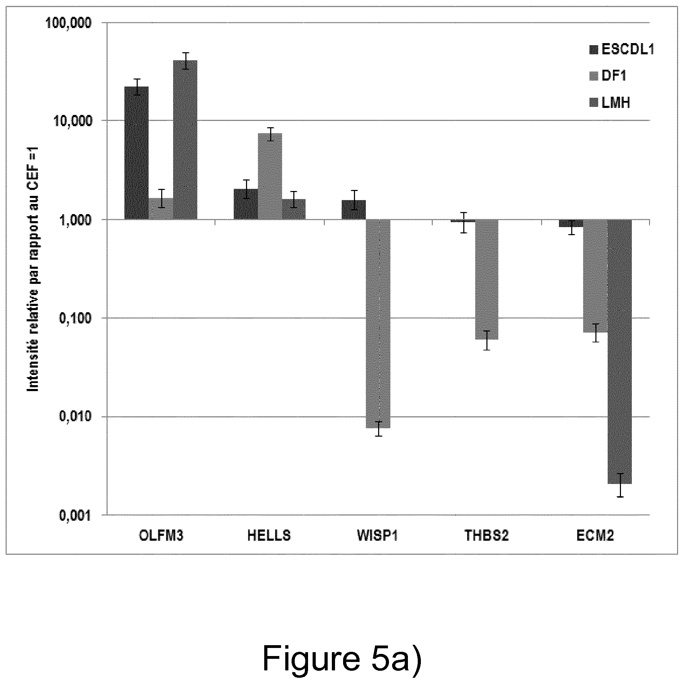

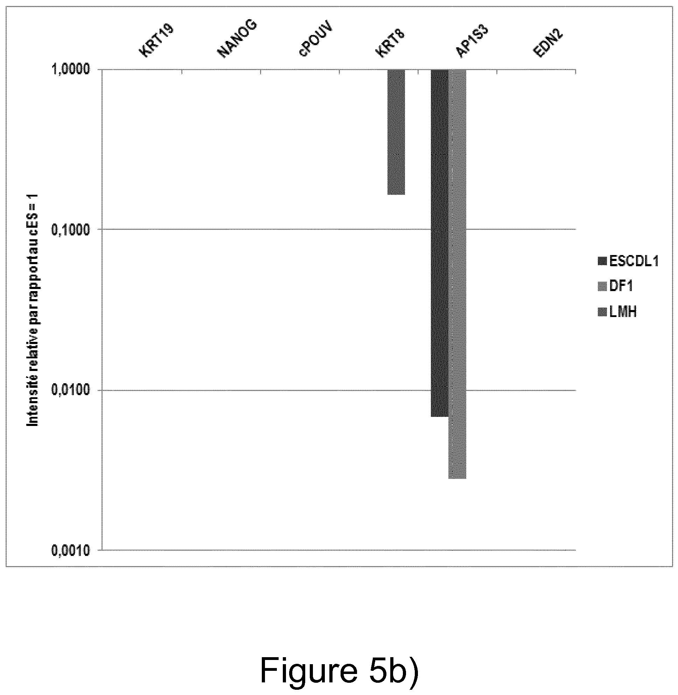

En particulier, cette lignée cellulaire exprime les marqueurs suivants : CD44⁺, intégrine beta1, et collagène 1. En revanche elle n'exprime plus les antigènes reconnus par les anticorps IXF1, MC-480 (SSEA1) et EMA-1 alors que ces derniers sont exprimés par les cellules souches embryonnaires initiales. Elles n'expriment plus les gènes cPOUV/OCT4, NANOG, KRT19 et KRT8 mais expriment fortement le gène OLFM3 et les gènes WISP1, THBS2 et EDN2 à des niveaux comparables à ceux observés dans des fibroblastes primaires (pris égal à 1 dans la figure). Le niveau de ces gènes dans les cellules fibroblastiques DF1 est de 10 à 100 fois plus faible (Figure 5). De la même façon, les cellules hépatiques LMH ne présentent absolument pas le même profil d'expression de ces marqueurs. La présente invention est également relative à une utilisation de la lignée cellulaire décrite ci-dessus comme substrat pour la production *in vitro* de virus aviaires.

La présente invention est également relative à une utilisation de la lignée cellulaire telle que décrite ci-dessus pour titrer des virus aviaires, et ainsi déterminer l'importance de l'infection virale.

Enfin, la présente invention concerne également un procédé de préparation *in vitro* d'une préparation vaccinale, comprenant la mise en culture de la lignée telle que décrite ci-dessus et son infection par au moins un virus aviaire. Autrement dit, l'invention concerne un procédé de préparation *in vitro* d'une préparation vaccinale, comprenant au moins les étapes suivantes :
A) Mise en culture de cellules souches embryonnaires aviaires en présence d'un stroma pendant au moins 3 jours ;
B) Culture dans un milieu à faible concentration de sérum pendant au moins 2 jours ;
C) Culture dans un milieu à faible concentration de sérum comprenant entre 1 et 10 mM de hexaméthylène bisacétamide (HMBA), pendant au moins 2 jours ;
D) Culture dans un milieu à faible concentration de sérum pendant au moins 10 jours ;
E) Culture ou congélation d'une lignée cellulaire aviaire permettant la réplication de virus aviaires ;
F) Infection de la lignée cellulaire obtenue par au moins un virus aviaire.

La présente demande est également relative à une préparation vaccinale comprenant une lignée cellulaire telle que décrite ci-dessus, infectée par au moins un virus aviaire.
Dans le cadre des différentes utilisations spécifiées ci-dessus, les virus aviaires sont en particulier choisis parmi les Mardivirus et les Birnavirus.

### DESCRIPTION DES FIGURES

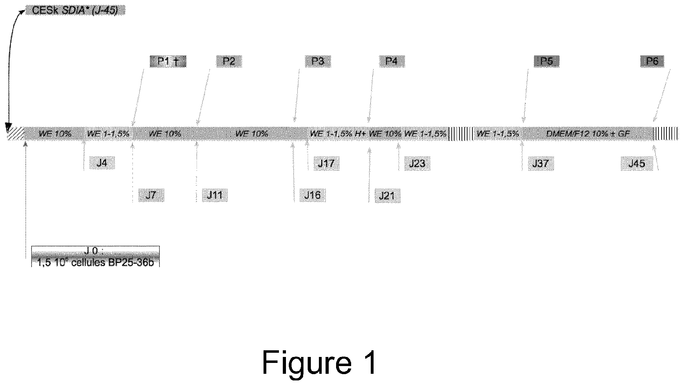

**Figure 1****.** Protocole d'établissement de la lignée ESCDL-1 : La période initiale d'induction des cellules BP25-36b est représentée entre les jours 0 et 45 par la barre centrale, montrant l'alternance des milieux utilisés pour la culture des cellules. Le décompte des passages est indiqué au-dessus, des passages 1 à 6 (P1 à P6). Le P1† correspond en fait à une trypsination courte destinée à éliminer les cellules mortes suivie d'une incubation en milieu à 10% SVF. Le décompte des jours par rapport à l'initiation de la culture figure en dessous de la barre centrale (J0 à 45). Les milieux indiqués sont le milieu E de William (WE) ou le DMEM/F12. Les concentrations 1-1,5% se rapportent à des concentrations en SVF (1%) et sérum de poulet (1,5%). L'abréviation GF correspond à « growth factors », tels qu'employés dans le milieu de croissance des cellules BP25 et décrits dans le matériel et méthode.
**Figure 2****.** Cellules ESCDL-1 : cellules au passage 52, en culture à basse (A) ou moyenne (B) densité 24 heures après division. La barre d'échelle représente 50 µm (microscopie en contraste de phase).
**Figure 3****.** Courbe de croissance des cellules ESCDL-1 entre les passages 25 et 30.
**Figure 4****.** Expression génique des marqueurs de cellules souches en RT-PCR : Oct4 (cPOUV) et NANOG.
**Figure 5**.a) Analyse de l'expression de gènes différentiellement exprimés entre les cellules ESCDL1, DF1 et LMH. Le niveau d'expression des gènes est mesuré par PCR en temps réel, le gène RSP17 étant pris comme référence comme décrit précédemment (Lavial et al, 2007). Le niveau relatif d'expression est pris égal à 1 dans le type cellulaire des CEF, fibroblastes embryonnaires primaires de poulet.b) analyse de l'expression de gènes plus spécifiquement exprimés dans les cellules souches embryonnaires (cES). Le niveau d'expression dans ces cellules est pris égal à 1. Les niveaux dans les cellules ESCDL1, DF1 et LMH sont très inférieurs (entre 1000 et 10 000 fois moins exprimé) ou même indétectable dans certain cas (Nanog par ex).

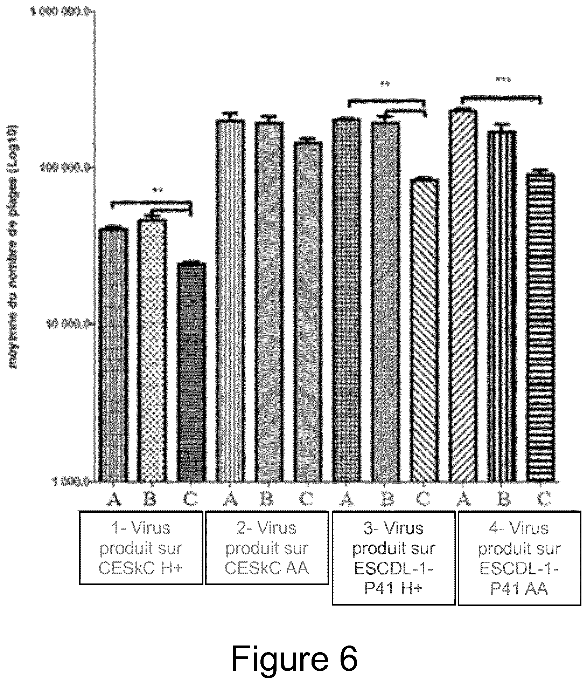

**Figure 6****.** Réplication des Mardivirus : Comparaison de la production virale (B20UL17mRFP) sur les cellules primaires (1 & 2) avec celle obtenue sur les ESCDL-1 P41 (3 & 4). Les titrages ont été effectués sur des cellules primaires (A), des ESCDL-1 aux passages 43 (B) et 98 (C). H+ : HMBA 5mM ; AA : Acide Ascorbique 0,3mM.
**Figure 7****.** Dissémination virale - analyse de la taille des plages induites par la multiplication du virus sur des cellules primaires (CESkC) et des cellules de la lignée ESCDL-1.

### EXEMPLES

### Matériels et Méthodes

1. Facteurs de croissance, produits chimiques et anticorps
   Les facteurs de croissance (PeproTech - France) pour la croissance des cellules souches embryonnaires aviaires ont été utilisés aux concentrations suivantes :
   ▪ IGF-1 (PeproTech-100-11) à 5 ng/ml
   ▪ IL6 (PeproTech-200-06) à 2 ng/ml
   ▪ sIL6-Ra (PeproTech-200-06R) à 2 ng/ml
   ▪ SCF (PeproTech-300-07) à 1 ng/ml

   Le HMBA [*N,N'*-Hexamethylene bis(acétamide) - ref. 224235], l'acide rétinoïque (ref. R2625), le DMSO (Dimethyl sulfoxyde - ref. D2650), le valproate (Valproic acid, sodium salt - ref. P4543) ont été achetés chez Sigma - Aldrich (Sigma Aldrich - France) et utilisés aux concentrations indiquées dans le texte.
2. Cellules
   a. Cellules souches BP25 : les cellules ont été entretenues dans les conditions décrites (Pain *et al.,* 1996), sur une couche nourricière (feeder-layer) de cellules STO irradiées, en milieu DMEM/F12 (1 :1) (Gibco- Invitrogen - France) additionné de 10% de sérum foetal de veau (SVF) (PAN - D. Dutscher - France), de pyruvate de Sodium (1 mM - Lonza - France), de glutamine (2mM - Lonza - France) et d'acides aminés non essentiels (NEAA 100X - Lonza - France) et contenant les facteurs de croissance aux concentrations indiquées au paragraphe précédent (milieu ES).
   b. Cellules ESCDL-1 : Les ESCDL-1 ont été obtenues par différenciation des BP25 (cf infra). Les cellules sont cultivées à 37°C, en atmosphère contenant 5% de CO₂, en milieu DMEM/F12 (1 :1) (Invitrogen - France) additionné de 10% de SVF (PAN - D. Dutscher - France), de pyruvate de Sodium (1 mM - Lonza - France), de glutamine (2mM - Lonza - France) et d'acides aminés non essentiels (NEAA 100X - Lonza - France). Les cellules sont trypsinées 2 fois par semaine (trypsine 10X - T4549 - Sigma Aldrich - France) et divisées selon un ratio de 1 pour 3.
   c. Cellules primaires de peau embryonnaire de poulet (CESkC) : les cellules primaires ont été obtenues selon le protocole décrit (Dorange *et al.,* 2000).
   d. Cellules 3867K et NWB : les lignées transformées par le virus RB-1B-UL47EGFP (Jarosinski *et al.,* 2012), obtenues dans le laboratoire de V. Nair, ont été cultivées en milieu RPMI 1640 (Lonza - France) contenant 10% de SVF, 10% de trypose phosphate broth (TPB) (ref T8782 - Sigma Aldrich - France) et 4,5 g/l de glucose. Les 2 lignées lymphocytaires transformées 3767K et NWB sont issues de tumeurs rénales prélevées sur des poulets infectés par le virus RB-1B-UL47EGFP (les lignées nous ont été données par Lorraine Smith - The Pirbright Institute - Compton, Berkshire, UK).
3. Virus
   a. GaHV-2 (MDV)
      i. Bac20 et virus dérivés : Les virus Bac20 (Schumacher *et al.,* 2000), Bac20eGFPVP22 (Denesvre *et al.,* 2007) et Bac20UL17-mRFP (Chbab *et al.,* 2009) ont été obtenus par transfection des bacmides contenant le génome viral parental (Bac20) ou muté.
      ii. RB-1B et virus dérivés: le virus RB-1B a été obtenu dans des conditions de transfections de bacmide similaires à celles décrites pour le Bac20, à partir du bacmide décrit par L. Petherbridge (Petherbridge *et al.,* 2004). Le virus RB-1B-UL17mRFP a été construit selon la même logique que le Bac20UL17-mRFP dans le contexte du bacmide RB-1B décrit par Jarosinski (Jarosinski *et al.,* 2007). Le virus RB-1B-UL47EGFP (Jarosinski *et al.,* 2012) a été obtenu par mise en contact des cellules testées avec les cellules 3867K ou NWB.
   b. MeHV1 : la souche vaccinale FC-126 a été passée en culture à partir d'une préparation vaccinale lyophilisée (Lyomarex® - Mérial - France). Tous les inoculum des Mardivirus (GaHV-2 et MeHV1) sont constitués de cellules infectées, congelées et conservées en azote liquide
   c. GaHV-1 (ILTV) : le virus utilisé correspond à la souche vaccinale Nobilis ® (Intervet - France). Le virus a été produit sur cellules LMH à partir de la préparation vaccinale lyophilisée. Les inoculum viraux ont été conservés à - 80°C après centrifugation à 200xg des surnageants provenant des cellules infectées (Fuchs & Mettenleiter, 1999).
   d. IBDV : le birnavirus de la bursite infectieuse du poulet, souche vaccinale CT, nous a été donné sous forme de surnageant de culture infectée par B. Delmas (Lejal *et al.,* 2000).
4. Différenciation des cellules souches
   a. Exposition aux agents cytodifférenciants : les cellules BP25 en phase exponentielle de croissance ont été trypsinées, dénombrées et ensemencées à 1 10⁵ cellules/cm² en boites de culture prétraitées par une solution de gélatine bovine (gélatine bovine à 0,1% en PBS - ref. G9391 Sigma - France). Après 24 heures à 37°C en milieu E de William contenant 10% de sérum foetal de veau, les cellules ont été incubées 24 à 48 heures dans le même milieu contenant 3% de sérum de poulet (SP) (Gibco- InvitroGen - France) et 2% de sérum de veau foetal (SVF). Les tapis confluents ont été exposés pendant 24 heures aux différentes molécules cytodifférenciantes en milieu E de William contenant 1% de SVF et 1,5% de SP (WE 1-1,5%) puis infectés (cf infra).
   b. ESCDL-1 : Les cellules BP25 au passage 36 ont été ensemencées à 1,5 10⁶ cellules dans une boite de 75 cm² contenant une matrice extracellulaire préparée à partir de cellules primaires (CESkC) suivant un protocole adapté de la publication de C. Coraux (Coraux *et al.,* 2003). Les cellules ont été d'abord cultivées en milieu WE 10% SF pendant 4 jours (Figure 1) puis incubées en milieu WE 1% SVF-1,5% SP pendant 3 jours avec un changement de milieu. Après trypsination les cellules ont été remises en culture dans une boite de 75cm², en WE contenant 10% de SVF durant 10 jours, trypsinées et exposées à une induction par le HMBA (5mM) en WE 1% SVF-1,5% SP durant 72 heures (ESCDL-1 Passage 3). Les cellules ont été trypsinées, ensemencées sur support de culture gélatiné et cultivées en WE 10% SVF durant 1 passage (P4). Les cellules ont été ensuite incubées pendant 15 jours en WE 1% SVF-1,5% SP trypsinées et cultivées en milieu DMEM-F12 contenant 10% de SF (P5 à P7). A partir du passage 7 les cellules ont été trypsinées 2 fois par semaine, divisées suivant un ratio de 1 pour 3, et cultivées jusqu'au passage 18 dans le même milieu. Les cellules ont été conservées en azote liquide à partir de ce passage (P18).
5. Transfection : les cellules ESCDL-1 ont été transfectées en utilisant la technologie Amaxa™ (Amaxa Nucleofector - Lonza - France), avec le kit Basic Fibroblast (ref VPI-1002) et des quantités d'ADN plasmidique ou bacmidique variant de 4 à 8 µg pour 4 10⁶ cellules. Après transfection les cellules ont été distribuées dans 3 puits de plaque de culture à 6 puits, préalablement gélatinés, et cultivées pendant 16 heures en milieu DMEM/F12 contenant 10% de SVF. Les cellules ont été placées dans un milieu en adéquation avec l'expérimentation (cf infra). Pour la sélection de lignées stables exprimant des transgènes sous promoteur pCMV en pCDNA3, les cellules ont été placées en milieu de sélection, DMEM/F12 10% SVF contenant 0,5 mg/ml de G418 (Ref - Sigma Aldrich) à la suite de la première trypsination 24 à 48 heures après la transfection. En absence de gène de sélection sur le plasmide codant pour le transgène, ce dernier a été co-transfecté avec un plasmide apportant un gène de résistance à l'hygromycine (pTK-HYG2). Dans ce cas les cellules ont été placées en milieu sélectif (DMEM/F12 10%SVF avec 80µg/ml d'hygromycine) dès 24 heures après la transfection.
6. Infections virales
   a. Préparation des inoculum
      i. Transfection de bacmides : dans les cellules ESCDL-1 les bacmides contenant les génomes viraux ont été transfectés à l'aide de la technologie Amaxa. Dans les CESC BP25 induites, les bacmides ont été tranfectés à l'aide du réactif FuGENE (Roche) en respectant les instructions du fabricant. Après transfection les cellules ESCDL-1 ont été incubées en milieu DMEM/F12 10% SVF durant 16 heures puis en milieu DMEM/F12 contenant 1% de SVF, 1,5% de SP et 0,3mM d'acide ascorbique. Les cellules BP25 induites ont été replacées en WE 1-1,5% avec les inducteurs aux concentrations décrites.
      ii. Utilisation de cellules infectées triées : la préparation des cellules infectées triées a été décrite (Denesvre *et al.,* 2007). Après incubation pendant 1h30 à 2 heures à 37°C, les inoculum (cellules triées) sont éliminés et les cellules ESCDL-1 sont replacées en milieu DMEM/F12 contenant 1% de SVF, 1,5% de SP et 0,3mM d'acide ascorbique. Les cellules BP25 induites sont remises en WE 1-1,5% avec les inducteurs aux concentrations décrites.
      iii. Utilisation de lymphocytes transformés : les cellules NWB ou 3867K ont été centrifugées, placées en milieu basse concentration en sérum foetal (DMEM/F12) et ensemencées sur un tapis confluent de cellules ESCDL-1. Après une incubation de 1h30 à 37°C l'inoculum a été éliminé et le milieu remplacé par du milieu DMEM/F12 contenant 1% de SVF, 1,5% de SP et 0,3mM d'acide ascorbique.
      iv. Congélation des inoculum : dans tous les cas les inoculum sont préparés à partir de tapis de cellules infectées présentant un effet cytopathique maximal. Après trypsination et centrifugation (200xg pendant 10 minutes). Les culots cellulaires ont été repris dans un milieu de congélation composé de 95% SVF et 5% DMSO et les cellules réparties en cryotubes selon un ratio de 3 tubes / boite de 75cm2. Après 24 heures en boite isotherme à -80°C les tubes ont été transférés en azote liquide.
   b. Titrages : Les titrages ont été réalisés sur des tapis cellulaires 48h après ensemencement, sur des cellules primaires (CESkC) ou des cellules ESCDL-1. La méthode décrite antérieurement (Blondeau *et al.,* 2008) a été adaptée au contexte des cellules ESCDL-1 en ce que le milieu DMEM/F12 a été substitué au WE et que, après inoculation, les tapis cellulaires ont été placés sous milieu semi-solide composé de DMEM/F12 ou de WE contenant 1% de méthylcellulose (Méthylcellulose ref 25 449.182 - VWR-Prolabo), 1% SVF, 1,5% SP et 3 mM acide ascorbique (ESCDL-1). Après 3 jours (Bac20 et mutants) ou 4 jours (RB-1B et mutants) les tapis cellulaires ont été fixés par incubation en présence d'une solution de paraformaldhédyde (ref P6148 - Sigma Aldrich - France) à 4% en tampon PBS durant 20 minutes à température ambiante. La solution de fixation a été ensuite éliminée, et les tapis rincés en PBS. Pour la détection des antigènes viraux les cellules ont été perméabilisées et la réaction d'immunofluorescence a été réalisée selon un protocole décrit (Dorange *et al.,* 2000). La mesure des tailles de plages dans différents contextes cellulaires a été réalisée selon le protocole décrit par N. Richerioux (Richerioux *et al.,* 2012), sur un nombre moyen de 80 plages individualisées.
   c. Charge virale : Le nombre de copies de génome viral par cellule a été estimé par utilisation d'une réaction de PCR quantitative (qPCR) de type Taqman, selon le protocole décrit par K. Jarosinski (Jarosinski *et al.,* 2002). Brièvement, le gène cellulaire iNOS et le gène viral ICP4 sont quantifiés dans les échantillons à l'aide d'un couple d'amorces et d'une sonde spécifique pour chaque gène. (Tableau 2). Afin de quantifier les mesures en nombre de copies, une gamme de dilution avec un nombre connu de copies d'ADN de chaque gène cible a été utilisée. Le standard iNOS est un plasmide pBS-iNOS (don de K. Jarosinski) ajusté à une concentration de 5 ng/µL, stocké à -20°C. Le standard ICP4 est un bacmide BAC-20 ajusté à 50 ng/µL, stocké à 4°C. Les gammes de dilution des standards sont faites de 10⁻³ à 10⁻⁷ en eau. Les qPCR ont été effectuées en plaque 96 puits avec le kit Absolute-Blue qPCR Mix&ROX (ABgene), les couples de primers ainsi que les sondes (Eurogentec). Les réactions ont été faites dans un volume final de 20 µL (10 µL de Mix Fast Blue 2X + 0,10 µL de chaque primer à 100 µM + 0,5 µL de sonde à 10 µM + 9,5 µL d'ADN).
7. Détection des antigènes cellulaires et viraux.
   a. Immunofluorescence : Les cellules ont été cultivées sur lamelle de verre de 14mm de diamètre et 0,17mm d'épaisseur (Marienfeld - VWR - France), en plaque de 24 puits (ref 353935 BD Falcon - France) après que les lamelles aient été incubées en gélatine à 0,1% en PBS durant 30 minutes. Les cellules ont été fixées en paraformaldéhyde à 4%. Les conditions de réalisation de la réaction d'immunofluorescence sur des cellules fixées et perméabilisées ont été décrites (Chbab *et al.,* 2009). Sur les cellules non perméabilisées, l'ensemble des incubations a été effectué en tampon PBS contenant 2% d'albumine sérique bovine (BSA) (bovine serum albumin, fraction V - PAA - France). Les anticorps primaires utilisés sont décrits dans le tableau 1. Les anticorps secondaires utilisés, anti IgG de souris, anti IgG de Lapin ou anti IgG de poule, sont couplés à l'Alexa 488 ou 594 (Molecular Probes - Invitrogen - France). L'actine polymérisée (F-actin) a été marquée par la phalloidine couplée à l'Alexa 594 (Ref A12381 - Molecular Probes - Invitrogen - France). Les photographies ont été prises à l'aide un microscope Zeiss Axiovert 200M ®, équipé d'un Colibri II ®, d'un dispositif Apotome ® Zeiss d'une caméra AxioCam MRm Zeiss ® et piloté par le logiciel Axiovision (Carl Zeiss SA - France).
   b. Immunoempreinte (WB). Les conditions dans lesquelles les antigènes viraux ont été détectés à partir d'extraits de cellules ESCDL-1 ont été les mêmes que celles décrites pour les cellules primaires (Chbab *et al.,* 2009).
8. Microscopie électronique à transmission : les cellules ESCDL-1 au passage 25 ont été infectées par un virus Bac20 au passage 3 après transfection sur ces mêmes cellules. Les cellules ont été cultivées sur une plaque 6 puits UpCell (Nunc UpCell surface Ref 174901 - Thermo Scientific - France) dans les conditions décrites (cf supra), infectées (ou « mock » infectées pour les cellules contrôles) et les tapis ont été récupérés selon le protocole décrit par le fabricant pour être ensuite fixés dans le fixateur de Trump. Les cellules infectées ou non infectées ont été préparées pour l'examen en microscopie électronique selon un protocole décrit antérieurement (Denesvre *et al.,* 2007).

### RÉSULTATS

### Exemple 1. Isolement de la lignée ESCDL-1

L'établissement d'une lignée permissive à partir des cellules BP25 a été conduit selon une logique d'induction reposant sur l'exposition successive à des matrices extracellulaires complexes dérivant de cellules permissives (Stromal cell-Derived Inducing Activity - SDIA -(Kawasaki et al., 2000)) et à l'action cytodifférenciante du HMBA. La préparation des matrices extracellulaires à partir des cellules primaires de peau de poulet a été effectuée selon le protocole décrit par Coraux (Coraux *et al.,* 2003). Les supports de culture obtenus ont été stockés 45 jours à 4°C et l'absence de croissance cellulaire a été vérifiée au terme de ce stockage. Au jour 0 de l'induction, les cellules souches ont été ensemencées sur une boite de Pétri contenant la matrice extracellulaire, à raison de 26 500 cellules / cm², en milieu WE contenant 10% de SVF (Figure 1). Après un cycle d'exposition à des concentrations réduites en sérum et 2 trypsinations, les cellules au passage 3 ont été exposées pendant 3 jours à l'action du HMBA (5mM), en milieu WE contenant 1% de SVF et 1,5% de SP. La suite du protocole d'établissement (Figure 1) s'est déroulée sur une durée de 25 jours, a comporté 3 trypsinations aboutissant au passage 6 après établissement de la culture. Les cellules ont alors été transférées en milieu DMEM/F12 contenant 10% de SVF, milieu dans lequel elles ont été ensuite cultivées. Le rythme de trypsination est passé de 1 par semaine (passages 6 et 7) à 2 par semaine pour les passages 8 et suivants, avec un ensemencement moyen de 45 000 cellules par cm². En parallèle à l'établissement de la lignée, la permissivité pour la réplication virale a été testée de façon ponctuelle au passage 13, révélant une multiplication du virus Bac20 sur les cellules ESCDL-1. La lignée a été considérée comme établie à partir du passage 23 et des stocks de cellules congelées ont été constitués à partir du passage 18.

### Exemple 2. Caractérisation de la lignée ESCDL-1

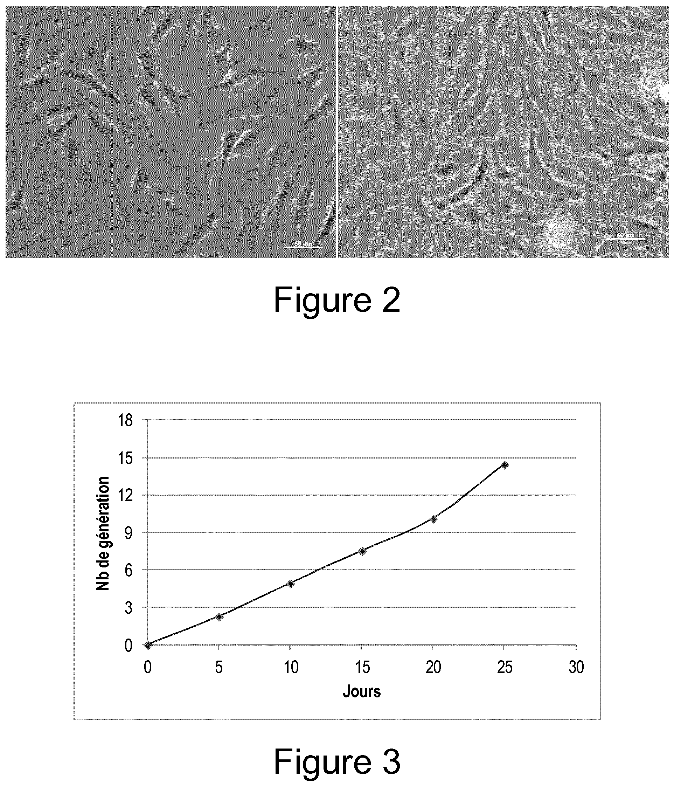

Les cellules ESCDL-1 possèdent une morphologie plutôt fibroblastique (Figure 2), leur temps de doublement est d'environ 40 heures et elles semblent montrer une perte progressive de leur potentiel de prolifération à partir du 100^{ème} passage. Leur courbe de croissance est montrée en figure 3.

La filiation entre BP25-36b et ESCDL-1 a été vérifiée de façon à établir que les cellules ne dérivaient pas de la persistance de cellules vivantes parmi les cellules ayant servi à établir la matrice extracellulaire. Les BP25 ayant été obtenues à partir d'embryons de poulet cou-nu (Pain *et al.,* 1996), nous avons utilisé une PCR différenciant les animaux LD1 des cou-nus (Mou *et al.,* 2011) sur des ADN génomiques extraits des cellules souches (BP25), des ESCDL-1 (P38) et des cellules primaires de peau embryonnaires (poules LD1). L'analyse des fragments obtenus a montré que les BP25 comme les ESCDL-1 dérivaient d'un embryon de génotype Cou-Nu (hétérozygote - Na/na) alors que les cellules primaires étaient issues d'un génotype « wt » (homozygote na/na).

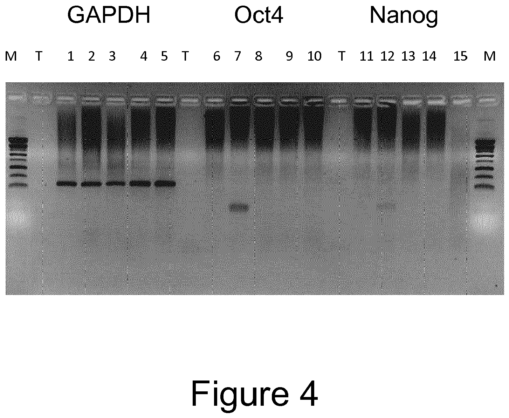

La figure 4 représente la détection des ARN messagers des marqueurs Oct4 et NANOG dans les cellules BP25 et ESCDL-1, par la technique RT PCR. Seules les cellules souches BP25 expriment les ARNm des marqueurs OCT4 (7) et NANOG (12). Ces ARNm sont indétectables dans les ESCDL-1 (puits 10 et 15). La détection des ARNm de la GAPDH dans toutes les préparations d'ARN (1 à 5) témoigne de la qualité égale des préparations d'ARNm de peau (1-6-11), cellules souches BP25 (2-7-12), foie (3-8-13), rein (4-9-14) et cellules ESCDL-1 (5-10-15). Ces résultats ont été confirmés en qRT PCR. En immunofluorescence, il s'est avéré impossible de visualiser l'expression d'autres marqueurs de cellules souches dans les ESCDL-1 (Tableau 1).

**Tableau 1 : Comparaison de l'expression des marqueurs de pluripotence sur les cellules BP25 et les ESCDL-1 (les cellules MDCK ont été utilisées comme contrôle). Les antigènes membranaires SSEA-1 (caractéristique des cellules ES) et EMA-1 (considéré comme un bon marqueur de cellules PGC) ont été détectés sur les cellules BP25 par immunofluorescence indirecte alors que les ESCDL-1 ne présentaient aucun marquage.**

| | | Cellules (non perméabilisées) | | |
|---|---|---|---|---|
| Anticorps | Spécificité | ESCDL-1 P39 | BP25 | MDCK P22 |
| IXF1 | Epithelial StemCell Marker | - | + | - |
| MC480 | SSEA-1 | - | + | - |
| EMA-1 | PGC marker | - | + | - |
| TROMA-1 | CytoK-EndoA | - | - | + †? |

Enfin l'activité télomérase a été mesurée dans les ESCDL-1 en comparaison avec les cellules LMH (témoin positif) et d'autres lignées cellulaires (DF1 et CLEC) et cellules primaires (CES kC) ; une faible activité peut être détectée, comparable à celle observée pour les cellules DF1 et CLEC (Esnault *et al.,* 2011), mais considérablement inférieure à celle observée dans les cellules LMH.

Une analyse comparative par RT-PCR (Lavial et al, 2007) de l'expression de gènes de pluripotence (5a) et de gènes marqueurs des fibroblastes (5b) entre les cellules DF1, LMH, CEF, cES (BP25) et ESCDL-1 obtenues selon l'invention a été réalisée quant à la présence de certains marqueurs. Les amorces utilisées pour cette analyse sont listées dans le Tableau 2 ci-dessous. Les résultats de cette analyse sont regroupés sur la figure 5 a et 5b.

**Tableau 2**

| **Nom du gène** | **Gene ID** | **Sens** | **AntiSens** |
|---|---|---|---|
| AP1 S3 | 424807 | TGCGGTGAAAGCCATGGAAGAC | GCAGGTGTGTACAGCAGTTCTCTT |
| | | | TCC |
| cPOUV/OCT 4 | 427781 | TGCAATGCAGAGCAAGTGCTGG | ACTGGGCTTCACACATTTGCGG |
| ECM2 | 415953 | GTCAGAAATTTGCTTTAACCACAC AAG | TTTATCCAAGCCAAAGGTGCTATT C |
| EDN2 | 419559 | CTGGAGCCCAAGGCAGACGC | GGCCAGTGATGCGGGCACTT |
| HELLS | 423750 | AAGCTCTGCTGGCAACCTGTGTC | AGGAACTGCTTGGCACTGTGTCG |
| KRT19 | 395861 | GGCTTCGGTGGTGGCTATGG | AGGACGCGAGGCGGTCATTC |
| KRT8 | 426896 | CCGGCAGCTGCGTGAGTACC | CGTGGTCCGGGTGTGGATGC |
| NANOG | 10027216 6 | TGCACACCAGGCTTACAGCAGTG | TGCTGGGTGTTGCAGCTTGTTC |
| OLFM3 | 424461 | TGGGCAGGAACCAACCACGTT | GTGCAAGCACCCGCCCAGTA |
| RSP17 | 374053 | ACACCCGTCTGGGCAACGAC | CCCGCTGGATGCGCTTCATC |
| THBS2 | 414837 | GTGGAAGCAGGTCACTCAAAC | CCCCAGTACCAGTTGTTGAATTC |
| WISP1 | 420322 | AGCCCAACTGCAAATACAACTG | TGAGTTTGTGCACATGGGAATG |

### Exemple 3. Permissivité des cellules ESCDL-1 pour la réplication des virus aviaires

### Les virus étudiés sont les suivants :

1. Virus atténués adaptés à la culture de cellule :
   ▪ Virus Bac20 et dérivés : le virus Bac20 UL17mRFP (Chbab *et al.,* 2009) a servi d'outil principal d'étude de la permissivité, en raison de la facilité de sa manipulation (protéine fluorescente rouge fusionnée à la protéine UL17) et de la conservation de ses caractéristiques de multiplication par rapport au virus parental (Bac20 non étiqueté). Les bacmides codant pour les virus Bac20 et Bac20EGFPUL49 (Blondeau *et al.,* 2008) ont également été transfectés dans les cellules ESCDL-1. L'infection avec les cellules infectées (cellules primaires de peau de poulet) par le virus Bac20-eGFPUL49 triées a également été utilisée (Denesvre *et al.,* 2007)
   ▪ Virus MeHV-1 : le virus MeHV-1 souche FC126 a été inoculé sur les cellules ESCDL-1 à partir d'une préparation vaccinale lyophilisée (pas de cellules primaires).
2. Virus pathogènes : virus dérivant des Bacmides RB-1B.
   ▪ Virus RB-1B et RB-1BUL17mRFP : transfection de bacmides dans les cellules ESCDL-1
   ▪ Virus RB-1B UL47GFP : transmission de l'infection virale à partir de cellules T transformées (NWB ou 3867K) isolées d'animaux infectés par le virus décrit par Jarosinsky et coll. (Jarosinski *et al.,* 2012)

### Exemple 3A. Bac 20 UL17mRFP

Les essais ont été réalisés dans un premier temps avec des virus adaptés à la culture de cellule, dérivant du bacmide codant pour le génome du virus Bac20. La transfection d'un bacmide dans les cellules ESCDL-1 offre un modèle d'étude différent de celui des cellules primaires.

### Passages sériés et titrages comparatifs sur cellules primaires et ESCDL-1 :

**Tableau 3**

| Passage | Split ratio* | Durée Incubation | Nombre de tubes congelés | Titre (UFP/ml) | |
|---|---|---|---|---|---|
| | | | | CESkC† | ESCDL-1 |
| T1** | 5,4µg Bacmide/4,5 10⁶ cellules | 7 jours | | | 250 UFP° |
| P1 | 1/6 | 5 | 3 | ND | ND |
| P2 | 1/5 | 4 | 4 | (2,6±0,79)*10⁵ | (2,12±0,71)x10⁵ |
| P3 | 1/20 | 6 | 3 | (3,4±0,79)*10⁵ | (3,05±0,79)x10⁵ |
| P4* | 1/20 | 6 | 3 | | (1,09±0,4)x10⁵ |
| P5 | 1/10 | 5 | 6 | | (1,16±0,09)x10⁶ |
| P6 | 1/30 | 5 | 6 | (5,5±0,8)*10⁴ | (5,2±0,8)x10⁴ |

| | | | | | |
|---|---|---|---|---|---|
| † : *CESkC : cellules primaires de peau embryonnaire de poulet* P4* : *passage auquel la charge virale a été mesurée dans les 2 systèmes de production (CESkC ou ESCDL-1)* | | | | | |

La réplication du virus Bac20 UL17mRFP en ESCDL-1 aboutit à l'obtention de titres infectieux de l'ordre de 1 à 5x10⁵ UFP/ml dès le deuxième passage du virus dans ce système cellulaire.

La charge virale (nombre de copies de génomes viraux/cellule) mesurée au passage 4 était équivalente dans les 2 systèmes de production (3 à 8x10³ copies de génome viral par cellule pour les CESkC et 2,4 à 10x10³ pour les ESCDL-1)

### Bac20 UL17mRFP : Progénie virale de titre équivalent sur les CEPP et sur les ESCDL-1 dans différentes conditions de milieu de culture des cellules

Les résultats sont présentés en figure 5 : Les ESCDL-1 présentent une capacité à répliquer le virus équivalente à celle des cellules primaires (CES kC).

### Bac20 UL17mRFP : Dissémination virale équivalente sur les CESkC et sur les ESCDL-1

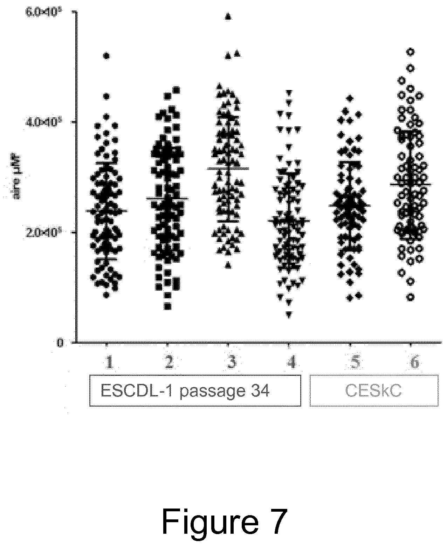

Les résultats sont présentés en figure 7. L'analyse de la taille des plages induites par la multiplication du virus sur les CESkC ou sur les ESCDL-1 montre une distribution et une taille équivalente pour un même inoculum dans les 2 systèmes cellulaires, quelles que soient les conditions de milieu testées.

### Exemple 3B. Bac20 EGFP-UL49 et Bac20

La transfection de Bacmides dans les cellules ESCDL-1 permet de réisoler des virus réplicatifs et se traduit par l'apparition d'un effet cytopathogène (plages de cellules infectées) dès le 4eme jour après la transfection.

Le virus Bac20 obtenu à partir de la transfection du bacmide se réplique dans les cellules ESCDL-1 et les étapes principales de la morphogénèse virale restent similaires à celles décrite dans les CESkC.

Comme montré dans le tableau ci-dessous, les cellules ESCDL-1 infectées par le virus Bac20 présentent des capsides intranucléaires de type A, B et C, des capsides C intracytoplasmiques et des capsides enveloppées entre les membranes nucléaires internes et externes.

**Tableau 4. Analyse de la répartition des capsides dans les cellules infectées :**

| Capsides A+B+C intranucléair es | Capsides périnuclé aires | Capsides non enveloppées intracytoplasmiques | Capsides tégumentées intracytoplasmiques | Capsides enveloppées intracytoplasmiques | Total |
|---|---|---|---|---|---|
| 656 | 54 | 356 | 14 | 4 | 1084 |
| 60,52% | 4,98% | 32,84% | 1,29% | 0,37% | 100% |

Le décompte de 1084 capsides dans 20 cellules ESCDL-1 permet toutefois de mettre en évidence une répartition légèrement différente de celle décrite pour les CESkC, tenant sans doute à la différence de conception de l'expérimentation. Il est cependant remarquable de constater que les pourcentages de capsides enveloppées intracytoplasmiques (capsides matures) sont identiques dans les 2 modèles cellulaires (Denesvre et al., 2007).

### Exemple 3C. BacRB-1B :

Dans le cadre du développement d'une lignée permissive à la réplication des Mardivirus, il était important de vérifier que cette lignée permettait la réplication de virus pleinement pathogènes, dérivant des BacRB-1B (Petherbridge *et al.,* 2004). La transfection de bacmide codant pour le virus RB-1B étiqueté par la mRFP sur la protéine UL17 s'est traduit par l'apparition d'un effet cytopathogène (ECP) viral dès le 4^{ème} jour après la transfection. Après 3 passages consécutifs en ESCDL-1, ce virus a été titré sur CESkC et sur ESCDL-1 ; les titres calculés sont similaires (2,5 à 3,5x 10⁵ UFP/ml) et dans la moyenne couramment observée pour ce type de virus produit sur des cellules primaires (substrat cellulaire « standard »). Nous avons également testé les possibilités de transmission de l'infection virale à partir de cellules lymphoïdes NWB et 3867K. La co-culture de cellules transformées réactivant le virus RB-1B UL47GFP avec les cellules ESCDL-1 aboutit à l'infection des cellules et au passage du virus en cycle lytique dans les ESCDL-1.

### Exemple 3D. Virus MeHV1 :

Le virus a été inoculé sur les cellules ESCDL-1 dans les conditions indiquées et cette inoculation s'est traduite par l'apparition d'un effet cytopathogène. Le virus résultant de ce premier passage a été titré sur CESkC et ESCDL-1. Les titres viraux, mesurés en UFP, sont identiques (4,05 ± 0,9 x10⁴).

### Exemple 3E : Birnavirus

Le virus IBDV de la souche CT (souche CT, numéro d'accès GENBANK EMBL AJ310185) adaptée à la culture de cellule a été produit soit en CEPP soit en ESCDL-1 et la progénie virale titrée en dilution limite dans les 2 systèmes cellulaires. Les titres obtenus sont de 1x10^{6,79} TCID50 sur les CESkC et de 1x10^{6,77} TCID50 sur les ESCDL-1

Le résultat du titrage des virus IBDV produits sur les cellules primaires (IBDV-PP1H+ ou H-) ou sur cellules ESCDL-1 (IBDV-ESCDL-1 34 ou 69H+ ou H-) sur les cellules secondaires (PP2) ou sur les ESCDL-1 au passage 39 est présenté dans le tableau 5 ci-dessous :

| Virus IBDV produit sur | Cellules utilisées pour le titrage | |
|---|---|---|
| | CESkC (P2)† | ESCDL-1 P39 |
| CESkC | 1x10^{6,79} | 1x10^{7,03} |
| ESCDL-1 P34 | 1x10^{6,77} | 1x10^{6,77} |
| ESCDL-1 P69 | 1x10^{6,65} | 1x10^{6,53} |

| | | |
|---|---|---|
| *† : CESkC P2 : cellules secondaires obtenues par trypsination et remise en culture des CESkC* | | |

Ce tableau donne une idée de la stabilité du phénotype des ESCDL-1 qui s'avèrent aussi sensibles à l'infection par l'IBDV aux passages 34 ou 69. Les titres viraux (calculés selon la méthode de Reed et Muench) sont similaires entre les CESkC et les ESCDL-1.

### Exemple 4 : Transfection de la lignée ESCDL-1

### Exemple 4A - ESCDL-1 Venus : lignée exprimant une EYFP

Les cellules ESCDL-1 au passage 21 ont été co-transfectées par le plasmide pCS2Venus et le plasmide pTK-Hyg2 (résistance à l'hygromycine) dans un rapport molaire de 20 molécules de pCS2Venus/1 molécule de pTK-Hyg2. Après transfection (Nucleofector Amaxa - kit Basic Fibroblast - programme F024) les cellules ont été sélectionnées en hygromycine (80µg/ml). Les ESCDL-1_Venus obtenues après clonage expriment fortement la protéine fluorescente, qui se localise dans le cytoplasme et le noyau, et répliquent les Mardivirus.

Les cellules ESCDL-1_Venus peuvent ainsi servir de substrat à l'étude des conditions de transmission de l'infection virale (transmission de cellule à cellule).

### Exemple 4B. ESCDL-1 UL37 : lignée transcomplémentante pour un gène viral essentiel.

Nous avons produit plusieurs bacmides codant pour des virus comportant des mutations invalidant un gène essentiel, UL37, codant une protéine de tégument. Les mutations concernent la totalité du gène (délétion) ou 2 codons codant pour 2 leucines participantes d'un motif « pseudo leucine zipper », motif conservé dans de nombreux orthologues d'UL37 (gène conservé chez tous les Herpesvirus). Des lignées ESCDL-1 transfectées par un pCDNA3-UL37 (ESCDL-1_37) ou pCDNA3-UL37-mut (ESCDL-1_37mut) ont été sélectionnées et testées pour leur capacité à répliquer les virus mutants et parentaux à partir des bacmides transfectés. Nous avons pu observer la formation de plages et la genèse d'une progénie virale pour les 2 virus mutés (délétion et mutation ponctuelle) sur les seules ESCDL-1_37. Ni les ESCDL-1 ni les ESCDL-1_37mut exprimant la protéine mutée ne peuvent complémenter les virus mutants.

Dans le contexte scientifique actuel, la lignée ESCDL-1 apparaît être la seule lignée « utilisable » pour ce genre d'étude, les cellules QM7 décrites précédemment (Schumacher et al., 2002) ne présentant pas une permissivité équivalente pour l'ensemble des Mardivirus.

### Exemple 4C. ESCDL-1 Lc3-GFP et LBR-GFP : clones cellulaires exprimant des protéines cellulaires fusionnées à la EGFP (Enhanced Green Fluorescent Protein)

Différents clones cellulaires ESCDL-1 exprimant les protéines Lc3-EGFP (marqueur d'autophagie) et LBR-EGFP (récepteur de la lamine B) ont été générés. Grâce à une expression stable du transgène, ces lignées permettent d'étudier la réponse cellulaire (l'autophagie et la déstabilisation de la lamina nucléaire) lors de processus pathologiques en particulier au cours d'infections par des virus ADN et ARN (par ex. les influenza aviaires capables d'activer les processus autophagiques). L'intérêt majeur de ces lignées stables est de suivre en temps réel la relocalisation des protéines Lc3 et LBR dans la cellule au cours d'infection.

Les ESCDL-1 exprimant une recombinase, la Flippase, ont également été obtenues ; l'expression du transgène a été établie et l'activité Flippase est en cours de caractérisation.

De par la facilité et la rapidité d'établissement des lignées ESCDL-1 exprimant des protéines cellulaires étiquetées (environ 1 mois), l'extension de ce système à un panel plus large de protéines peut être envisagée.

### REFERENCES

### BREVETS

WO 2007/110343
WO 98/006824
EP 2 572 728
EP 0 775 743

### NON BREVETS

Blondeau, Marc, Courvoisier, Vautherot & Denesvre (2008). Functional homologies between avian and human alpha-herpesvirus VP22 proteins in cell-to-cell spreading as revealed by a new cis-complementation assay. J Virol 82, 9278-9282.
Chbab, Chabanne-Vautherot, Francineau, Osterrieder, Denesvre & Vautherot (2009). The Marek's disease virus (MDV) protein encoded by the UL17 ortholog is essential for virus growth. Vet Res 40, 28.
Churchill (1968). Herpes-type virus isolated in cell culture from tumors of chickens with Marek's disease. I. Studies in cell culture. J Natl Cancer Inst 41, 939-950.
Coraux, Hilmi, Rouleau, Spadafora, Hinnrasky, Ortonne, Dani & Aberdam (2003). Reconstituted skin from murine embryonic stem cells. Curr Biol 13, 849-853.
Correia, Anisimov, Li & Brundin (2008). Growth Factors and Feeder Cells Promote Differentiation of Human Embryonic Stem Cells into Dopaminergic Neurons: A Novel Role for Fibroblast Growth Factor-20. Front Neurosci, 2, 26-34.
Denesvre, Blondeau, Lemesle, Le Vern, Vautherot, Roingeard & Vautherot (2007). Morphogenesis of a highly replicative EGFPVP22 recombinant Marek's disease virus in cell culture. J Virol 81, 12348-12359.
Dorange, El Mehdaoui, Pichon, Coursaget & Vautherot (2000). Marek's disease virus (MDV) homologues of herpes simplex virus type 1 UL49 (VP22) and UL48 (VP16) genes: high-level expression and characterization of MDV-1 VP22 and VP16. J Gen Virol 81, 2219-2230.
Esnault, Bonsergent, Larcher, Bed'hom, Vautherot, Delaleu, Guigand, Soubieux, Marc & Quéré (2011). A novel chicken lung epithelial cell line: Characterization and response to low pathogenicity avian influenza virus. Virus Res 159, 32-42.
Eyal-Giladi & Kovak (1976). From cleavage to primitive streak formation: a complementary normal table and a new look at the first stages of the development of the chick; I. General Morphology. Dev. Biol 49, 321-337.
Fuchs & Mettenleiter (1999). DNA sequence of the UL6 to UL20 genes of infectious laryngotracheitis virus and characterization of the UL10 gene product as a nonglycosylated and nonessential virion protein. J Gen Virol 80 (Pt 8), 2173-2182.
Geerligs, Quanz, Suurland, Spijkers, Rodenberg, Davelaar, Jongsma, Kumar (2008). Efficacy and safety of cell associated vaccines against Marek's disease virus grown in a continuous cell line from chickens. Vaccine. 26, 5595-5600.
Jarosinski, Arndt, Kaufer & Osterrieder (2012). Fluorescently tagged pUL47 of Marek's disease virus reveals differential tissue expression of the tegument protein in vivo. J Virol 86, 2428-2436.
Jarosinski, Margulis, Kamil, Spatz, Nair & Osterrieder (2007). Horizontal transmission of Marek's disease virus requires US2, the UL13 protein kinase, and gC. J Virol 81, 10575-10587.
Jarosinski, Yunis, O'Connell, Markowski-Grimsrud & Schat (2002). Influence of genetic resistance of the chicken and virulence of Marek's disease virus (MDV) on nitric oxide responses after MDV infection. Avian Dis 46, 636-649.
Kawaguchi, Nomura, Hirayama & Kitagawa (1987). Establishment and characterization of a chicken hepatocellular carcinoma cell line, LMH. Cancer Res, 47, 4460-4463.
Kawasaki, Mizuseki, Nishikawa, Kaneko, Kuwana, Nakanishi, Nishikawa & Sasai (2000). Induction of midbrain dopaminergic neurons from ES cells by stromal cell-derived inducing activity. Neuron 28, 31-40.
Lavial, Acloque, Bertocchini, Macleod, Boast, Bachelard, Montillet, Thenot, Sang, Stern, Samarut & Pain (2007). The Oct4 homologue PouV and Nanog regulate pluripotency in chicken embryonic stem cells. Development, 134, 3549-3563.
Lee, Foster, Bottje, Jang, Chandra, Gentles & Kong. (2013). Establishment of an immortal chicken embryo liver-derived cell line. Poult Sci. 92,1604-1612
Lejal, Da Costa, Huet & Delmas (2000). Role of Ser-652 and Lys-692 in the protease activity of infectious bursal disease virus VP4 and identification of its substrate cleavage sites. J Gen Virol 81, 983-992.
Marks & Rifkind (1989). Induced differentiation of erythroleukemia cells by hexamethylene bisacetamide: a model for cytodifferentiation of transformed cells. Environ Health Perspect. 80, 181-188.
Moscovici, Moscovici, Jimenez, Lai, Hayman & Vog (1977). Continuous tissue culture cell lines derived from chemically induced tumors of Japanese quail. Cell. 11, 95-103.
Mou, Pitel, Gourichon, Vignoles, Tzika, Tato, Yu, Burt, Bed'hom, Tixier-Boichard, Painter & Headon (2011). Cryptic patterning of avian skin confers a developmental facility for loss of neck feathering. PLoS Biol 9, e1001028.
Nakano , Kodama & Honjo (1994). Generation of lymphohematopoietic cells from embryonic stem cells in culture. Science 265, 1098-1101.
Ogura & Fujiwara.(1987). Establishment and characterization of a virus-free chick cell line. Acta Med Okayama. 41,141-143.
Pain, Clark, Shen, Nakazawa, Sakurai, Samarut & Etches (1996). Long-term in vitro culture and characterisation of avian embryonic stem cells with multiple morphogenetic potentialities. Development 122, 2339-2348.
Panacchia, Dellambra, Bondanza, Paterna, Maurelli, Paionni & Guerra. (2010). Nonirradiated human fibroblasts and irradiated 3T3-J2 murine fibroblasts as a feeder layer for keratinocyte growth and differentiation in vitro on a fibrin substrate. Cells Tissues Organs 191, 21-35.
Petherbridge, Brown, Baigent, Howes, Sacco, Osterrieder & Nair (2004). Oncogenicity of virulent Marek's disease virus cloned as bacterial artificial chromosomes. J Virol 78, 13376-13380.
Preston & McFarlane (1998). Cytodifferentiating agents affect the replication of herpes simplex virus type 1 in the absence of functional VP16. Virology 249, 418-426.
Richerioux, Blondeau, Wiedemann, Remy, Vautherot & Denesvre (2012). Rho-ROCK and Rac-PAK Signaling Pathways Have Opposing Effects on the Cell-to-Cell Spread of Marek's Disease Virus. PLoS One 7, e44072.
Schumacher, Tischer, Fuchs & Osterrieder (2000). Reconstitution of Marek's disease virus serotype 1 (MDV-1) from DNA cloned as a bacterial artificial chromosome and characterization of a glycoprotein B-negative MDV-1 mutant. J Virol 74, 11088-11098.
Schumacher, Tischer, Teifke, Wink & Osterrieder (2002). Generation of a permanent cell line that supports efficient growth of Marek's disease virus (MDV) by constitutive expression of MDV glycoprotein E. J Gen Virol 83, 1987-1992.
Solomon, Witter, Nazerian, Burmester (1968). Studies on the Etiology of Marek's Disease. I. Propagation of the Agent in Cell Culture. Exp Biol Med 127, 173-177
Storlie, Jackson, Hutchinson & Grose (2006). Delayed biosynthesis of varicella-zoster virus glycoprotein C: upregulation by hexamethylene bisacetamide and retinoic acid treatment of infected cells. J Virol 80, 9544-9556.

## Revendications

1. Procédé d'obtention d'une lignée cellulaire aviaire non transformée permettant la réplication de virus aviaires *in vitro,* comprenant au moins les étapes suivantes :
a) Mise en culture de cellules souches embryonnaires aviaires en présence d'un stroma pendant au moins 3 jours ;
b) Culture dans un milieu à faible concentration de sérum pendant au moins 2 jours ;
c) Culture dans un milieu à faible concentration de sérum comprenant entre 1 et 10 mM de hexaméthylène bisacétamide (HMBA), pendant au moins 2 jours ;
d) Culture dans un milieu à faible concentration de sérum pendant au moins 10 jours ;
e) Culture ou congélation d'une lignée cellulaire aviaire permettant la réplication de virus aviaires.

2. Procédé selon la revendication 1, **caractérisé en ce que** les virus aviaires sont des Mardivirus ou des Birnavirus.

3. Procédé selon l'une quelconque des revendications 1 ou 2, **caractérisé en ce que** les cellules souches embryonnaires aviaires sont issues de poulet.

4. Procédé selon l'une quelconque des revendications 1 à 3, **caractérisé en ce que** les cellules souches embryonnaires aviaires sont isolées à partir de blastoderme aux stades X à XIV.

5. Procédé selon l'une quelconque des revendications 1 à 4, **caractérisé en ce qu'**après les étapes b), c) et d), le procédé comprend de plus des étapes de culture des cellules dans un milieu de croissance approprié.

6. Procédé selon l'une quelconque des revendications 1 à 5, **caractérisé en ce que** la lignée cellulaire aviaire obtenue a un phénotype stable pendant au moins 60 passages successifs dans un milieu de culture approprié.

7. Procédé selon l'une quelconque des revendications 1 à 6, **caractérisé en ce que** la lignée cellulaire aviaire obtenue est modifiée génétiquement pour exprimer des protéines d'intérêt.

8. Lignée cellulaire susceptible d'être obtenue par le procédé selon l'une quelconque des revendications 1 à 7, **caractérisée en ce qu'**elle exprime les marqueurs suivants : CD44+, intégrine beta1, collagène 1 et OLFM3.

9. Lignée cellulaire selon la revendication 8, ladite lignée n'exprimant pas les marqueurs suivants : cPOUV/OCT4, nanog, SSEA1, EMA1, IXF1, KRT8 et KRT19.

10. Utilisation de la lignée cellulaire selon l'une des revendications 8 ou 9 comme substrat pour la production *in vitro* de virus aviaires.

11. Utilisation de la lignée cellulaire selon l'une des revendications 8 ou 9 pour titrer des virus aviaires.

12. Procédé de préparation *in vitro* d'une préparation vaccinale, comprenant la mise en culture de la lignée cellulaire selon l'une des revendications 8 ou 9 et son infection par au moins un virus aviaire.

13. Préparation vaccinale comprenant une lignée cellulaire selon l'une des revendications 8 ou 9, infectée par au moins un virus aviaire.

## Patentansprüche

1. Verfahren zwecks Erhalts einer nicht transformierten Vogelzelllinie, die die *in-vitro-*Replikation von Vogelviren erlaubt, umfassend mindestens die folgenden Schritte:
a) Kultivieren von embryonalen Vogelstammzellen in Gegenwart eines Stromas während mindestens 3 Tagen;
b) Kultur in einem Milieu mit schwacher Serumkonzentration während mindestens 2 Tagen;
c) Kultur in einem Milieu mit schwacher Serumkonzentration, umfassend zwischen 1 und 10 mM Hexamethylenbisacetamid (HMBA) während mindestens 2 Tagen;
d) Kultur in einem Milieu mit schwacher Serumkonzentration während mindestens 10 Tagen;
e) Kultur oder Kongelation einer Vogelzelllinie, die die Replikation von Vogelviren erlaubt.

2. Verfahren nach Anspruch 1, **dadurch gekennzeichnet, dass** die Vogelviren Mardiviren oder Birnaviren sind.

3. Verfahren nach einem der Ansprüche 1 oder 2, **dadurch gekennzeichnet, dass** die embryonalen Vogelstammzellen vom Huhn stammen.

4. Verfahren nach einem der Ansprüche 1 bis 3, **dadurch gekennzeichnet, dass** die embryonalen Vogelstammzellen aus dem Blastoderm in den Stadien X bis XIV isoliert wurden.

5. Verfahren nach einem der Ansprüche 1 bis 4, **dadurch gekennzeichnet, dass** nach den Schritten b), c) und d) das Verfahren zusätzlich Kulturschritte der Zellen in einem geeigneten Wachstumsmilieu umfasst.

6. Verfahren nach einem der Ansprüche 1 bis 5, **dadurch gekennzeichnet, dass** die erhaltene Vogelzelllinie während mindestens 60 aufeinanderfolgenden Durchgängen in einem geeigneten Kulturmilieu einen stabilen Phänotyp hat.

7. Verfahren nach einem der Ansprüche 1 bis 6, **dadurch gekennzeichnet, dass** die erhaltene Vogelzelllinie genetisch modifiziert ist, um interessierende Proteine zu exprimieren.

8. Zelllinie, die mit dem Verfahren nach einem der Ansprüche 1 bis 7 erhaltbar ist, **dadurch gekennzeichnet, dass** sie die folgenden Marker exprimiert: CD44+, Integrin Beta1, Collagen 1 und OLFM3.

9. Zelllinie nach Anspruch 8, wobei die Linie die folgenden Marker nicht exprimiert: cPOUV/OCT4, nanog, SSEA1, EMA1, IXF1, KRT8 und KRT19.

10. Verwendung der Zelllinie nach einem der Ansprüche 8 oder 9 als Substrat für die *in-vitro-*Produktion von Vogelviren.

11. Verwendung der Zelllinie nach einem der Ansprüche 8 oder 9 zum Titrieren von Vogelviren.

12. Verfahren für die Herstellung *in vitro* eines Impfpräparats, umfassend das Kultivieren der Zelllinie nach einem der Ansprüche 8 oder 9 und ihre Infektion durch mindestens einen Vogelvirus.

13. Impfpräparat, umfassend eine Zelllinie nach einem der Ansprüche 8 oder 9, die durch mindestens einen Vogelvirus infiziert ist.

## Claims

1. A method for obtaining a non-transformed avian cell line allowing replication of avian viruses in vitro, comprising at least the following steps:
a) cultivating avian embryonic stem cells in the presence of a stroma for at least 3 days;
b) cultivating in a medium with a low serum concentration for at least 2 days;
c) cultivating in a low serum concentration medium comprising between 1 and 10 mM of hexamethylene bisacetamide (HMBA), for at least 2 days;
d) cultivating in a low serum concentration medium for at least 10 days;
e) cultivating or freezing of an avian cell line allowing replication of avian viruses.

2. The method according to claim 1, **characterized in that** the avian viruses are Mardiviruses or Birnaviruses.

3. The method according to any one of claims 1 or 2, **characterized in that** the avian embryonic stem cells stem from chicken.

4. The method according to any one of claims 1 to 3, **characterized in that** the avian embryonic stem cells are isolated from blastoderms at stages X to XIV.

5. The method according to any one of claims 1 to 4, **characterized in that** after the steps b), c) and d), the method further comprises steps for cultivating cells in a suitable growth medium.

6. The method according to any one of claims 1 to 5, **characterized in that** the avian cell line obtained has a stable phenotype during at least 60 successive passages in a suitable culture medium.

7. The method according to any one of claims 1 to 6, **characterized in that** the avian cell line obtained is genetically modified for expressing proteins of interest.

8. A cell line which may be obtained by the method according to any one of claims 1 to 7, **characterized in that** it expresses the following markers: CD44+, beta1 integrin, collagen 1 and OLFM3.

9. The cell line according to claim 8, said line not expressing the following markers: cPOUV/OCT4, NANOG, SSEA1, EMA1, IXF1, KRT8 and KRT19.

10. Use of the cell line according to one of claims 8 or 9 as a substrate for producing in vitro avian viruses.

11. Use of the cell line according to one of claims 8 or 9 for titration of avian viruses.

12. A method for preparing in vitro a vaccinal preparation, comprising the cultivation of the cell line according to one of claims 8 or 9 and its infection with at least one avian virus.

13. A vaccinal preparation comprising a cell line according to one of claims 8 or 9, infected with at least one avian virus.


### SEQUENCE LISTING

<110> INSTITUT RATIONAL DE LA RECHERCHE AGRONOMIQUE
<120> PROCEDE DE SÉLECTION D'UNE LIGNE CELLULAIRE PERMISSIVE POUR LA REPLICATION DE VIRUS AVIAIRES
<130> 366041D32442
<150> FR 1357346
   <151> 2013-07-25
<160> 24
<170> PatentIn version 3.5
<210> 1
   <211> 22
   <212> DNA
   <213> artificial
<220>
   <223> Amorce sens du gène AP1S3
<400> 1
   tgcggtgaaa gccatggaag ac 22
<210> 2
   <211> 27
   <212> DNA
   <213> artificial
<220>
   <223> Amorce antisens du gène AP1S3
<400> 2
   gcaggtgtgt acagcagttc tctttcc 27
<210> 3
   <211> 22
   <212> DNA
   <213> artificial
<220>
   <223> Amorce sens du gène cPOUV/OCT4
<400> 3
   tgcaatgcag agcaagtgct gg 22
<210> 4
   <211> 22
   <212> DNA
   <213> artificial
<220>
   <223> Amorce antisens du gène cPOUV/OCT4
<400> 4
   actgggcttc acacatttgc gg 22
<210> 5
   <211> 27
   <212> DNA
   <213> artificial
<220>
   <223> Amorce sens du gène ECM2
<400> 5
   gtcagaaatt tgctttaacc acacaag 27
<210> 6
   <211> 25
   <212> DNA
   <213> artificial
<220>
   <223> Amorce antisens du gène ECM2
<400> 6
   tttatccaag ccaaaggtgc tattc 25
<210> 7
   <211> 20
   <212> DNA
   <213> artificial
<220>
   <223> Amorce sens du gène EDN2
<400> 7
   ctggagccca aggcagacgc 20
<210> 8
   <211> 20
   <212> DNA
   <213> artificial
<220>
   <223> Amorce antisens du gène EDN2
<400> 8
   ggccagtgat gcgggcactt 20
<210> 9
   <211> 23
   <212> DNA
   <213> artificial
<220>
   <223> Amorce sens du gène HELLS
<400> 9
   aagctctgct ggcaacctgt gtc 23
<210> 10
   <211> 23
   <212> DNA
   <213> artificial
<220>
   <223> Amorce antisens du gène HELLS
<400> 10
   aggaactgct tggcactgtg tcg 23
<210> 11
   <211> 20
   <212> DNA
   <213> artificial
<220>
   <223> Amorce sens du gène KRT19
<400> 11
   ggcttcggtg gtggctatgg 20
<210> 12
   <211> 20
   <212> DNA
   <213> artificial
<220>
   <223> Amorce antisens du gène KRT19
<400> 12
   aggacgcgag gcggtcattc 20
<210> 13
   <211> 20
   <212> DNA
   <213> artificial
<220>
   <223> Amorce sens du gène KRT8
<400> 13
   ccggcagctg cgtgagtacc 20
<210> 14
   <211> 20
   <212> DNA
   <213> artificial
<220>
   <223> Amorce antisens du gène KRT8
<400> 14
   cgtggtccgg gtgtggatgc 20
<210> 15
   <211> 23
   <212> DNA
   <213> artificial
<220>
   <223> Amorce sens du gène NANOG
<400> 15
   tgcacaccag gcttacagca gtg 23
<210> 16
   <211> 22
   <212> DNA
   <213> artificial
<220>
   <223> Amorce antisens du gène NANOG
<400> 16
   tgctgggtgt tgcagcttgt tc 22
<210> 17
   <211> 21
   <212> DNA
   <213> artificial
<220>
   <223> Amorce sens du gène OLFM3
<400> 17
   tgggcaggaa ccaaccacgt t 21
<210> 18
   <211> 20
   <212> DNA
   <213> artificial
<220>
   <223> Amorce antisens du gène OLFM3
<400> 18
   gtgcaagcac ccgcccagta 20
<210> 19
   <211> 20
   <212> DNA
   <213> artificial
<220>
   <223> Amorce sens du gène RSP17
<400> 19
   acacccgtct gggcaacgac 20
<210> 20
   <211> 20
   <212> DNA
   <213> artificial
<220>
   <223> Amorce antisens du gène RSP17
<400> 20
   cccgctggat gcgcttcatc 20
<210> 21
   <211> 21
   <212> DNA
   <213> artificial
<220>
   <223> Amorce sens du gène THBS2
<400> 21
   gtggaagcag gtcactcaaa c 21
<210> 22
   <211> 23
   <212> DNA
   <213> artificial
<220>
   <223> Amorce antisens du gène THBS2
<400> 22
   ccccagtacc agttgttgaa ttc 23
<210> 23
   <211> 22
   <212> DNA
   <213> artificial
<220>
   <223> Amorce sens du gène WISP1
<400> 23
   agcccaactg caaatacaac tg 22
<210> 24
   <211> 22
   <212> DNA
   <213> artificial
<220>
   <223> Amorce antisens du gène WISP1
<400> 24
   tgagtttgtg cacatgggaa tg 22